(19) Europäisches Patentamt
European Patent Office
Office européen des brevets

(11) **EP 2 097 089 B1**

(12) **EUROPÄISCHE PATENTSCHRIFT**

(45) Veröffentlichungstag und Bekanntmachung des Hinweises auf die Patenterteilung:
**17.08.2011 Patentblatt 2011/33**

(21) Anmeldenummer: **07817804.3**

(22) Anmeldetag: **19.11.2007**

(51) Int Cl.:
*A61K 36/61* (2006.01)    *A61P 19/08* (2006.01)
*A61P 19/02* (2006.01)    *A61P 19/10* (2006.01)

(86) Internationale Anmeldenummer:
**PCT/DE2007/002089**

(87) Internationale Veröffentlichungsnummer:
**WO 2008/061512 (29.05.2008 Gazette 2008/22)**

(54) **DIÄTETISCHES LEBENSMITTELS MIT ERHÖHTER ANTIINFLAMMATORISCHER UND FREIE RADIKALE BINDENDER WIRKUNG**

DIETETIC FOODSTUFF WITH AN IMPROVED ANTI-INFLAMMATORY AND FREE RADICAL BINDING ACTION

ALIMENT DIÉTÉTIQUE AYANT UN EFFET ANTI-INFLAMMATOIRE ACCRU ET UN EFFET ACCRU DE LIAISON AUX RADICAUX LIBRES

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL**

(30) Priorität: **21.11.2006 DE 102006055210**

(43) Veröffentlichungstag der Anmeldung:
**09.09.2009 Patentblatt 2009/37**

(73) Patentinhaber: **Ionescu, John G.**
**93485 Rimbach (DE)**

(72) Erfinder: **Ionescu, John G.**
**93485 Rimbach (DE)**

(74) Vertreter: **advotec.**
**Patent- und Rechtsanwälte**
**Bahnhofstrasse 2**
**94315 Straubing (DE)**

(56) Entgegenhaltungen:
**EP-A- 1 340 341        WO-A-01/47560**
**WO-A-03/037320        WO-A-2006/098625**
**WO-A-2007/070611        US-A1- 2002 197 352**
**US-A1- 2006 088 574**

• **DATABASE EPODOC EUROPEAN PATENT OFFICE, THE HAGUE, NL; XP002486357 & JP 02 265458 A (NIIKKEN FOOD KK) 30. Oktober 1990 (1990-10-30)**

**Beschreibung**

Die vorliegende Erfindung betrifft ein diätetisches Lebensmittel mit erhöhter antiinflammatorischer und freie Radikale bindender Wirkung.

Hintergrund der Erfindung:

Die Zahl der Allergien und chronischen Hauterkrankungen wie Neurodermitis, Psoriasis, chronische Ekzeme und Akne ist in den letzten 20 Jahren stark anges-tiegen. In Deutschland registriert man knapp 30 Mio. Allergiker (davon ca. 5 Mio. Neurodermitiskranke) und mehr als 4 Mio. Psoriasiskranke.

Bei der Neurodermitis handelt es sich um eine chronisch-rezidivierende Erkrankung, die meist im frühen Kindesalter beginnt und sich durch eine in den letzten Jahrzehnten ansteigende Prävalenz auszeichnet. Die auf Deutschland bezogenen Daten bewegen sich von ca. 10 % bis 20 % bei Kindern. Das klinische Bild variiert mit dem Lebensalter. Charakteristisch für das atopische Ekzem sind ein stark auftretender Juckreiz, Rötung, Schuppung, Nässen und Krustenbildung der Haut. Von den ekzemartigen Hautveränderungen sind am häufigsten Gelenkbeugen, Handrücken, Gesicht, Hals, Nacken und Brust betroffen. Die bisher bekannten Therapie beschränkten sich auf symptomatische Maßnahmen, da eine kausale Behandlung aufgrund der unklaren Pathogenese nicht möglich ist. Ätiologisch wird ein multifaktorielles Geschehen vermutet, so dass neben Störungen der Immuni-tät, des vegetativen Nervensystems und der Hautfunktion auch genetische und psychologische Faktoren eine Rolle spielen **[1]**.

Diese Faktoren scheinen so zusammenzuwirken, dass das Immunsystem mit dem Aufbau einer gänzlich unerwünschten T-Zellen-Produktion auf völlig harmlose Moleküle reagiert (z.B.: Hausstaubmilben, Pollen, verschiedene Nahrungsmittel etc.). Die Träger dieser Immunantwort sind TH-2 Zellen. Diese zeichnen sich durch ihre typische Zytokinsekretion (IL-4, IL-5, IL-13) aus und sind bei Patienten mit Neuro-dermitis für die Aktivierung der Plasmazellen im Sinne einer unkontrollierten IgE-Produktion, die als Marker dieser Erkrankungen sofortige Reaktionen (Typ I) gegen unterschiedliche Antigene vermittelt, verantwortlich.

In mehreren Untersuchungen konnte auch ein Zusammenhang zwischen der nutritiven Versorgung mit Spurenelementen und Vitaminen und dem Krankheitsbild der Neurodermitis festgestellt werden **[2, 3, 4]**. In einem Studienkollektiv von 116 Patienten wiesen 92% Lebensmittelintoleranzen auf. *Barth GA et al (2001)* konnten nachweisen, dass symptomatische Patienten signifikant weniger Milchprodukte, Fisch, Eier, Schweinefleisch, Obst und Nüsse verzehren als nichtsymptomatische. Die Studienergebnisse lassen den Schluss zu, dass eine Nährstoffsupplementation für den symptomatischen Patienten empfehlenswert ist **[5]**. Der umfassenden diätetischen Supplementation mit Mikronährstoffen kommt daher zunehmend Bedeutung zu.

Die Psoriasis vulgaris (1-3% der Weltbevölkerung sind betroffen) ist an einen ererbten Prädispositionsfaktor gebunden und durch (auto)-immunologisch mediierte Entzündungsprozesse in der Haut bedingt. Einerseits kommt es zur Dysregulation der Keratinozyten, andererseits akkumulieren aktivierte T-Zellen mit einem Zytokinsekretionsmuster (IL-2, TNF-$\alpha$, IFN-$\gamma$) das für so genannte TH-1-Zellen typisch ist. Entzündliche Prozesse, Infekte, Umweltschadstoffe, Medikamente sowie Verletzungen und psychosomatische Störungen begünstigen den Ausbruch der Krankheit.

Die Analyse des Therapiespektrums bei Psoriasis in den Industrieländern in den letzten 30 Jahren zeigt immer wieder dieselben Verfahren wie:

1. Die Bestrahlungstherapie: UV-Behandlung unter stationären oder ambulatorischen Bedingungen, oft kombiniert mit Chemotherapeutika wie Psoralen (PU-VA-Therapie)

2. Klima-Therapie in hohen Gebirgen oder am Meer kombiniert die positive Wirkung der Sonne (UV-Bestrahlung) mit der Mineral- und Spurenelementeinwirkung des Meerwassers. Auf dieser natürlichen Therapieform basieren auch kli-nische oder ambulante Ansätze mit UV-Strahlen und Sole-Therapie, die in Kliniken und Kurorten angeboten werden.

3. Lokale symptomatische Behandlung mit verschiedenen Salben, Cremes und Emulsionen ebenfalls in Hautkliniken und Praxen, meistens basierend auf Kortison-, Retinoid-, Salicylsäure-, Cignolin- und Teerformulierungen.

4. Innere Behandlung schwerster Psoriasisformen mit Vitamin-A-Derivaten (Tigason, Roaccutan) oder mit Zytostatika (Methotrexat, Cyclosporin).

Die oben erwähnten Therapieformen sind mit Sicherheit allen Psoriasis-Betroffenen längst bekannt und bis heute in jeder dermatologischen Therapiestätte auffindbar.

Alle diese Verfahren haben jedoch einige Nachteile wie z.B.:

ad 1. keines dieser Verfahren berücksichtigt wichtige Provokationsfaktoren psoriatischer Erscheinungen.

ad 2. Alle diese Verfahren richten sich lediglich auf die psoriatischen Symptome (Entzündungen und hohe Vermehrungsrate der Hautzellen, verbunden mit Schuppung), daher ist die beschwerdefreie Zeit begrenzt und die Rückfälle kommen nach wiederholter Behandlung immer schneller wieder.

ad 3. Alle diese Verfahren haben verschiedene Nebenwirkungen, die bei langfristiger Anwendung für den Patienten leichter oder gravierender sein können, von Hemmung der Immunreaktion über Atrophie der Haut, Ödeme, Haarverlust und innere Organschäden bis hin zu Hautkrebs.

Anhand dieser Tatsachen konzentriert sich die Hoffnung der Betroffenen in den letzten Jahren verstärkt auf neue, biologische, nebenwirkungsarme Therapieansätze, die unter Umständen auch eine längere Beschwerdefreiheit sichern können [6].

Neben einer genetischen Disposition im Einzelfall sieht die Mehrheit der Experten eine falsche Ernährung, die steigende Umweltbelastung und die assoziierten chronischen Infekte als Hauptursache für die rapid steigende Zahl der o.g. Erkrankungen [6, 7, 8, 9, 10, 11].

Als gemeinsamer Nenner in der Pathogenese diktieren die chronisch bzw. akuten inflammatorischen Zustände den klinischen Verlauf.

Hier besitzen die freien Radikate (Superoxid, Hydroxylradikal, Singulet-Sauerstoff bzw. Wasserstoff- und Lipidperoxide sowie die Stickstoffperoxide) eine wichtige Triggerfunktion für die Aktivierung der NF- kB-Transkriptionsfamilie und die Freisetzung der oben erwähnten inflammatorischen Zytokine [12].

Die Erfassung der freien Radikale in biologischen Humanproben ist deshalb von großer Bedeutung sowohl für die Auswertung des inflammatorischen Status als auch zur Überwachung des Therapieergebnisses.

Eine aktuelle Methodik, die lucigeninverstärkte ultraschwache Chemilumineszenz, vermittelt einen präzisen Einblick sowohl in die momentane Produktion freier Radikale in frischen biologischen Proben wie Blut, Plasma, Hautbiopsien, Haare, als auch in die gesamt-antioxidative Aktivität (AOA) des Plasmas und erlaubt die richtige Therapieauswahl [13, 14, 15]. Parallel durchgeführte Redoxpotentialmessungen in Blut oder Plasma ermöglichen eine rapide Auswertung der Redoxstörungen wie Oxidose (Verlust von Elektronen, Erhöhung des Sauerstoffpartialdrucks und der oxidativ wirkenden Äquivalenten wie z.B. Halogene, Schwermetalle und organische Stoffe in oxidierter Form) bzw. Redose (Akkumulation von Elektronen bzw. reduktiv wirkender Äquivalenten wie z.B. Zuckerarten, Thiolverbindungen, organische Stoffe in reduzierter Form, Sauerstoffmangel, etc.) metabolischer oder respiratorischer Natur) in den gleichen Proben [16, 17] sowie deren gezielte Behandlung. Normale Redoxverhältnisse sind essentiell für physiologische Signal-Rezeptor-Transduktionsfunktionen, Modulierung der NF-KB-Transkriptionsfamilie und der Genaktivität der enzymatischen Tätigkeiten, des Mitosezyklus und des Apoptoseprogramms in der Zelle.

Messverfahren:

Zur Erfassung der respiratorischen und metabolischen Oxidose- bzw. Redosezustände sowie des Normbereiches in Vollblut und Serum wurde in Zusammenarbeit mit dem Institut für Elektrochemie der Universität Erlangen der Prototyp einer temperierten Redoxzelle (37°C) für kleine biologische Proben (1-3 ml) unter $N_2$-Atmosphäre entwickelt. Die Eichung der ausgewählten Arbeits- und Referenzelektroden (Graphit- bzw. Platin-/ Iridium- vs. Ag/ AgCl-Elektroden) erfolgte mit einer Redox-Pufferlösung der Fa. Mettler Toledo, Deutschland. Durch Verbindung der Redox-Elektroden mit einem Potentiometer mit hohem Widerstand konnten die Potentiale (Eh in mV) gemessen werden. Für die kontinuierliche Darstellung der Potential/ Zeit-Kurven wurde das Potentiometer mit Hilfe eines Adapters mit einem PC verbunden. Die Stabilisierung der Redox-Kurven erfolgte nach ca. 6-8 Minuten. Gleichzeitig erfolgten pH-, $pO_2$- und $pCO_2$-Messungen mit einem AVL-Blutgasanalysator [17, 18].

Eine ultraschwache Photonenemission von Lichtspektren im Bereich von 180-800 nm existiert in allen lebenden Organismen und dieser Prozess ist eng verbunden mit dem oxidativen Stoffwechsel, der Erzeugung freier Radikale, Zellteilung, Zelltod, Photosynthese, vorzeitigem Altern, Karzinogenese und Wachstumsregulation Unter bestimmten Bedingungen, welche die Phagozytose und die Aktivierung der NADPH-Oxidase polymorphonuklearer Leukozyten (PMN) stimulieren, treten Lichtphotonen aus, die im Szintillationszähler gemessen werden können. In den letzten Dekaden wurden verschiedene Methoden zur Messung der Antioxidativen Aktivität (AOA) von biologischen Flüssigkeiten und Bestandteilen entwickelt. Einige benutzen eine Quelle zur Erzeugung Freier Radikale, ein Chemoluminiszenz (CL) Substrat (z.B. Luminol, Lucigenin), ein starkes Oxidationsmittel (z.B. Perborat), katalytische Enzyme wie Peroxidase, Xanthinoxidase und Verstärker wie Para-Iodophenol. Während der Oxidation geben Luminol und Lucigenin Lichtenergie ab, wenn die Elektronen in angeregtem Zustand zurück in den Grundzustand fallen. Die kontinuierliche Lichtabgabe spiegelt die Produktion der reaktiven $O_2$-Spezies (ROS) wider und reagiert empfindlich auf Unterbrechungen durch Radikalfänger. Die 3-Stufen-CL-Methode besteht darin, zu einer konstanten Menge von gepuffertem Lucigenin (bzw.

einer ROS erzeugenden Mischung beim AOA-Test) eine konstante Menge von Blut oder Serum zu geben, gefolgt von einer kurzen Präinkubation und Messung der Photonenzahlen über einen Zeitraum von 600 Sek. bei Raum- oder Körpertemperatur (22°C bzw. 37°C) [14, 15, 18].

Verschiebungen der beschriebenen Parameter (starke freie Radikalbildung bzw. Redosezustände in den zellulären Kompartimenten) sowie anderer immunbiologischer Parameter wurden bei Neurodermitis und Psoriasis bereits beschrieben in Folge der Aufnahme falscher Nahrungsmittel, persistierender Infekte und Schadstoffbelastung [6, 11, 17, 18, 19].

Diese Kausalitätstriade ist auch verantwortlich für den immer häufigeren Ausbruch allergischer Erkrankungen im Säuglingsalter. Hier spielen die von der Mutter auf den Fötus bzw. auf den Säugling übertragenen Allergene, Schadstoffe (Schwermetalle, Pestizide, Holzschutzmittel, etc.) und bei der Geburt übertragene Infekte der Vaginalflora die entscheidende Rolle beim Ausbruch allergischer Erkrankungen des Säuglings wie z.B. Milchschorf, Neurodermitis, allergisches Asthma oder Urtikaria [11, 19, 20].

Ein zusätzlicher Faktor ist die Ernährung des Säuglings, wobei die schadstoffbelastete Muttermilch oder die allergen- und zuckerreiche Flaschennahrung immer wieder eindeutige Provokationsfaktoren darstellen [19].

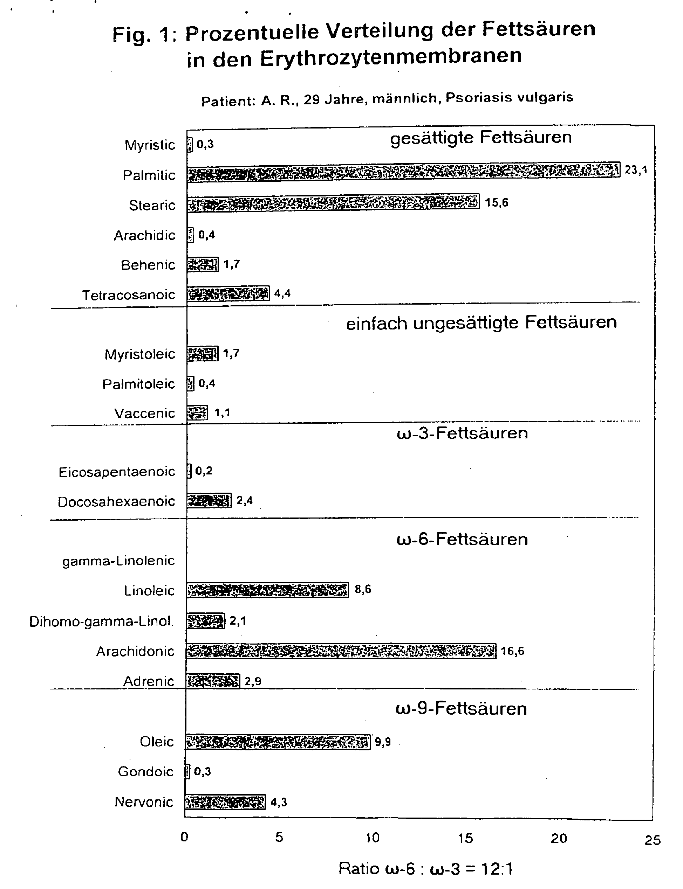

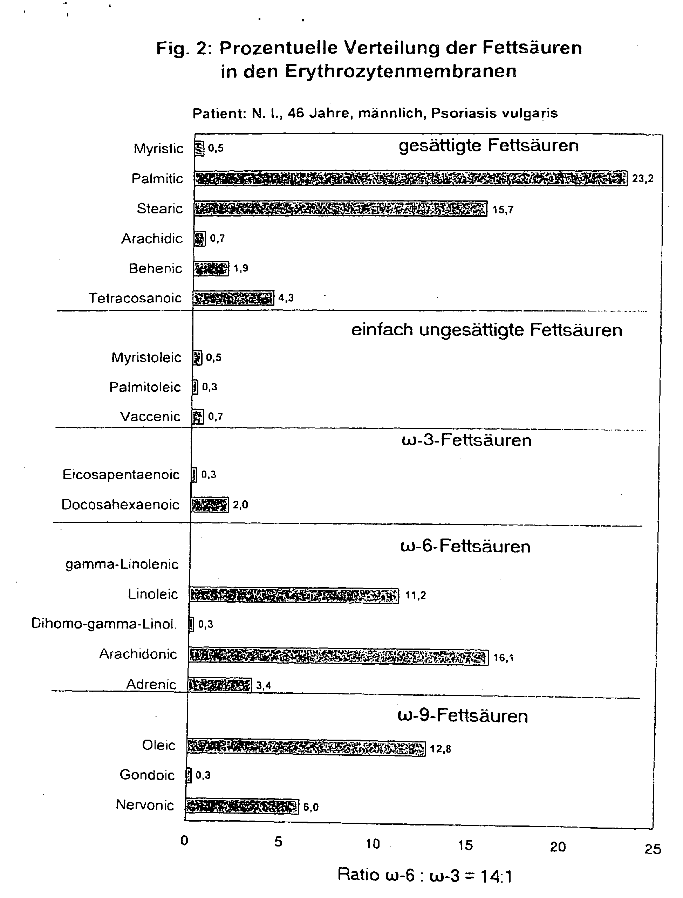

Sowohl die Muttermilch der neurodermitiskranken Frauen als auch die Serum-Phospholipide der Neurodermitis-Patienten weisen auf eine ungünstige Ratio der $\omega$-6: $\omega$-3 - Fettsäuren hin mit einem starken Überschuss der ersteren [21, 22]. Das o.g. Ungleichgewicht $\omega$-6 : $\omega$-3 wurde auch vom Erfinder wiederholt in der Phospholipidfraktion der Erythrozytenmembranen der Neurodermitiker und Psoriatiker mit Headspace-HPLC nachgewiesen; statt 3:1 werden Verhältnisse von 10:1 bis 20:1 gemessen. [Fig. 1 + 2].

Ein weiteres Merkmal atopischer Erkrankungen ist die chronische mikrobielle Fehlbesiedlung der Haut, der Schleimhäute und des Darmes mit grampositiven, gramnegativen und anaeroben Bakterien oder Sprosspilzen wie etwa Candida bei einer gleichzeitigen Dezimierung der physiologischen milchsäureproduzierenden Bakterien (Lactobacillus sp., Bifidobacterium sp., physiol. E. coli) [23, 24, 25, 26, 27].

Die falsche Darmbesiedlung ist auch die Ursache für die Zerstörung der Disaccharidasen (Laktase, Succrase, Maltase) mit chronischen Zuckerintoleranzen als direkte Folge und Malabsorptionsstörungen für geläufige Zuckerarten wie Laktose, Succrose, Maltose [24].

Die oben beschriebene Fehlbesiedlung des Darmes führt weiterhin zu einer chronisch erhöhten Darmdurchlässigkeit [28] und einer falschen Schulung der Lymphozyten aus den darmassoziierten Lymphoidstrukturen in Richtung allergische Steuerung (TH2-Antwort mit starkem IL4- und IL5-Zytokinmuster).

Der dadurch erhöhte Antigeneintritt (Nahrungs- und mikrobielle Antigene) in den Blutkreislauf führt zu einer chronisch erhöhten Bildung zirkulierender Immunkomplexe, die ihrerseits eine anhaltende Aktivierung des Gerinnungsprozesses mit peripheren Durchblutungsstörungen in den Kapillargefäßen bewirken [28, 29, 30]. Die chronisch kalten Extremitäten der meisten Hautpatienten sind ein Merkmal dieser Erkrankungen.

Gegenstand der Erfindung

Wie bekannt, stellen Nahrungsmittelintoleranzen bei Neurodermitikern, Asthmatikern, Psoriatikern und Umweltpatienten eine häufige Begleiterscheinung dar, die erheblich zur Unterhaltung der klinischen Symptome beitragen kann.

Die Nahrungsmittelallergien, besonders im Kindesalter, gehen mit chronisch entzündlichen Zuständen der Haut, der Schleimhäute und des Darmes einher und machen immer wieder die Ergebnisse geläufiger symptomatischer Cortison- oder Antihistaminikatherapien zunichte. Als Trigger aller entzündlicher Zustände steht primär eine starke Produktion freier Radikale, gefolgt von einer Freisetzung verschiedener Entzündungsmediatoren (Histamin, Prostaglandine, Leukotriene, Zytokine).

Intoleranzreaktionen bei Säuglingen und Kleinkindern mit allergischen Erkrankungen sind auf die ungünstige Zusammensetzung der Babynahrung / Flaschennahrung zurückzuführen aufgrund der starken Antigenpotenz geläufiger Kuhmilch- bzw. Sojaproteine und der hohen Anteile an Zuckerarten wie Saccharose, Laktose und Fruktose, die einerseits typische Intoleranzreaktionen hervorrufen können und andererseits die beste Nahrung für die chronisch gestörte Darmflora (pathogene Pilze und Bakterien) darstellen.

Auf dieser Grundlage bietet die Erfindung eine sachgerechte Alternative, die sowohl die starke Allergenpotenz der bisherigen Kuhmilch- bzw. Sojaproteine als auch die zuckervermittelten Intoleranzreaktionen bzw. mikrobielle Vergärungsprozesse im Darm ausschaltet.

Eine weitere Aufgabe der Erfindung besteht darin, ein diätetisches Lebensmittel mit verstärkter antiinflammatorischer und freie Radikale bindender Wirkung zur Verfügung zu stellen, welches zur diätetischen Behandlung , Neurodermitis, Psoriasis, Urticaria, Milchschord, allergisches Asthma.

Die genannten Aufgaben werden gelöst durch ein diätetisches Lebensmittel mit den Merkmalen des Anspruchs 1.

Das enthaltene Protein im erfindungsgemäßen Lebensmittel ist mindestens eines aus der Gruppe, bestehend aus Reisprotein, Weizenprotein, Erbsenprotein, Linsenprotein, Sojaprotein, Kartoffelprotein, Lupinenprotein, Molkeprotein, Stutenmilchprotein, Schafsmilchprotein, Ziegenmilchprotein und Collostrumprotein.

Mit Vorteil liegt das Protein im erfindungsgemäßen Lebensmittel aufgrund der hypoallergenen Eigenschaften in der hydrolysierten Form vor.

Der enthaltene Zucker im erfindungsgemäßen Lebensmittel ist vorzugsweise mindestens einer aus der Gruppe langsam verdaulicher Zuckeralkohole mit niedrigem glykämischen Index, bestehend aus Xylit, Mannit, Maltit, und Sorbit.

Gegenstand der Erfindung sind somit mehrere Kombinationen von hypoallergenen bzw. hydrolysierten Proteinen (Molke-, Stutenmilch-, Ziegenmilch-, Collostrum-, Reis-, Weizen-, Kartoffel-, Linsen-, Erbsenprotein) mit gärungsunfähigen Zucker-alkoholen bzw. Lactulose mit starker antioxidativer (freie Radikale bindender) Wirkung zur Behandlung bestimmter Erkrankungen.

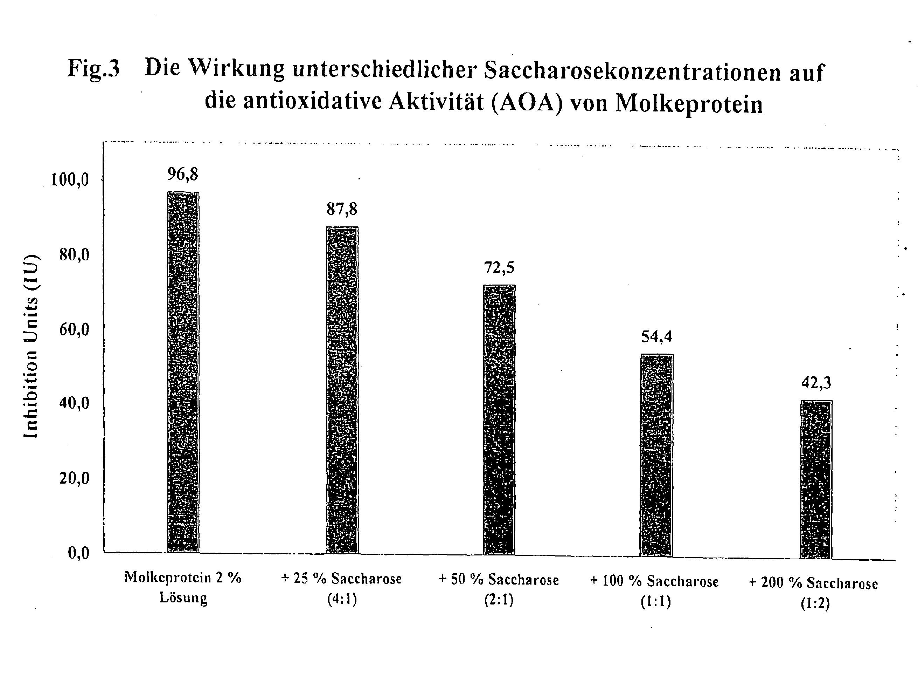

Zahlreiche Experimente des Erfinders haben nachgewiesen, dass die Kombination von ausgewählten hypoallergenen Proteinen mit guten antioxidativen Eigenschaften (AOA > 50 IU) mit höheren Mengen an "falschen" Zuckerarten wie z.B. Saccharose, Laktose, Galactose zu einer erheblichen Senkung der antioxidativen bzw. der antientzündlichen Aktivität führt [Fig. 3].

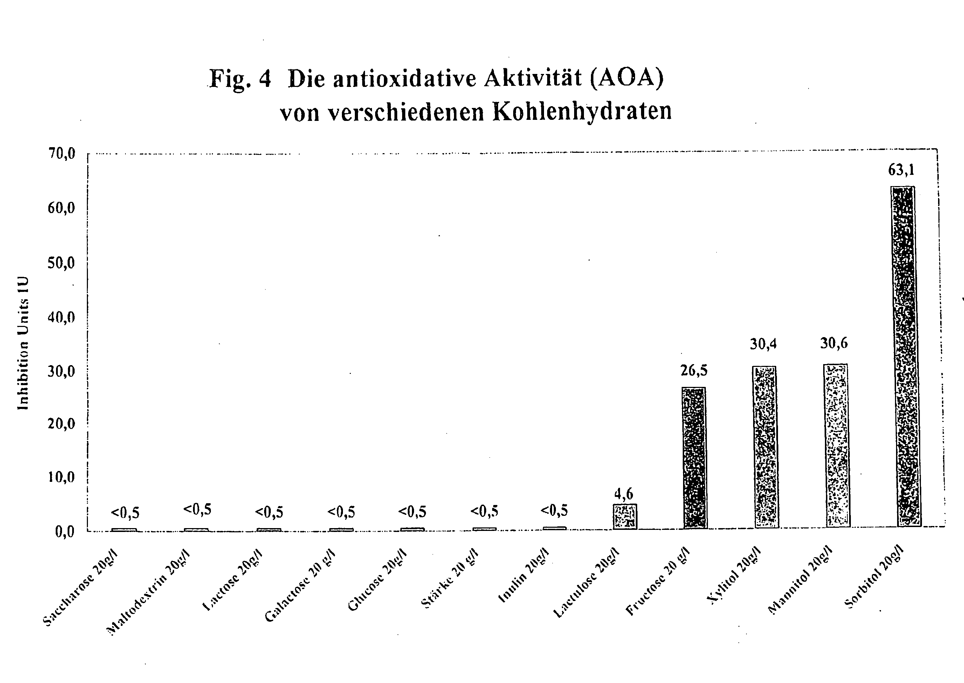

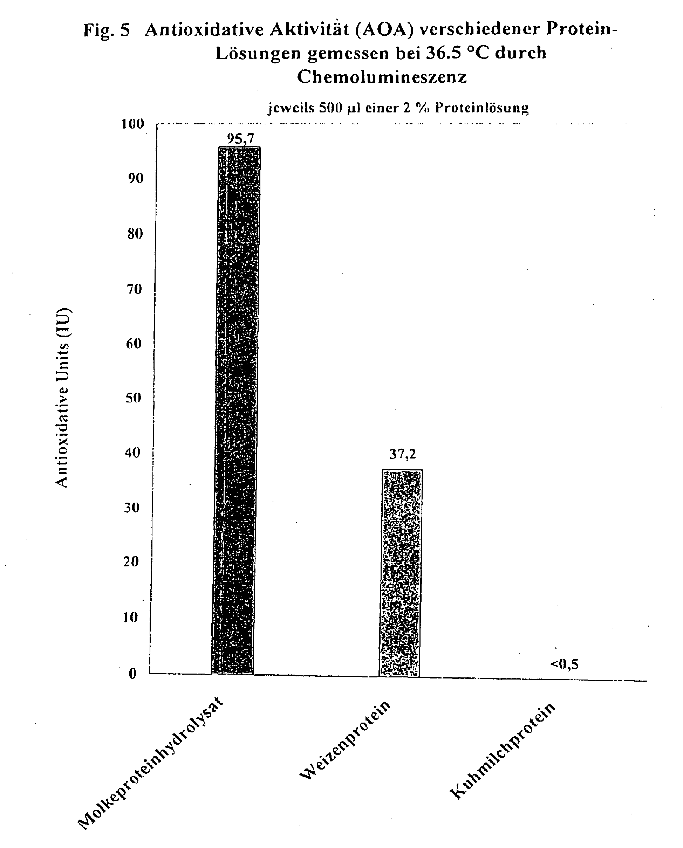

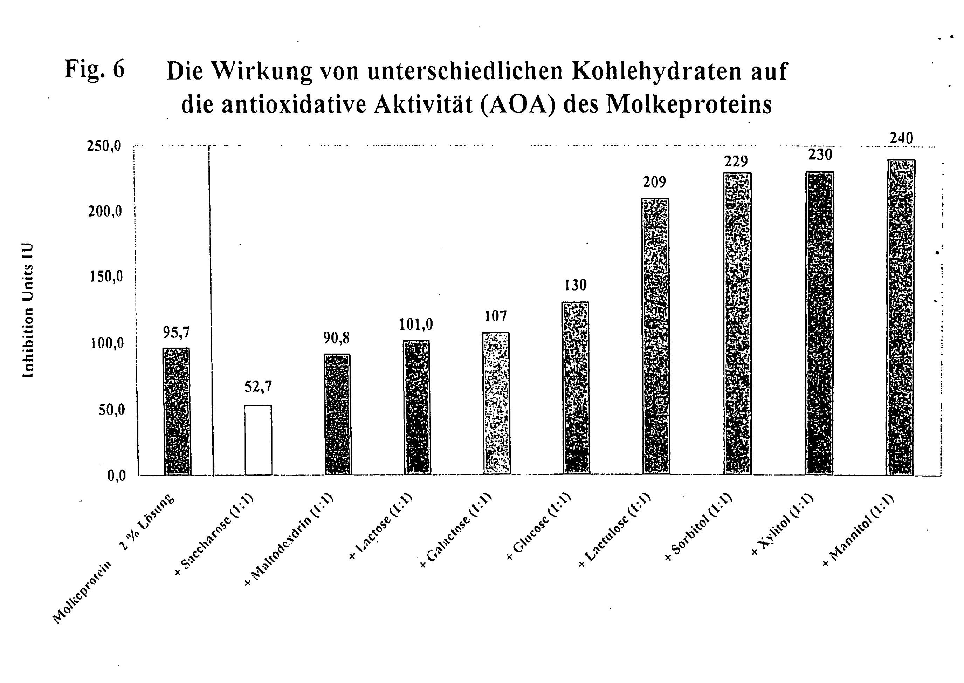

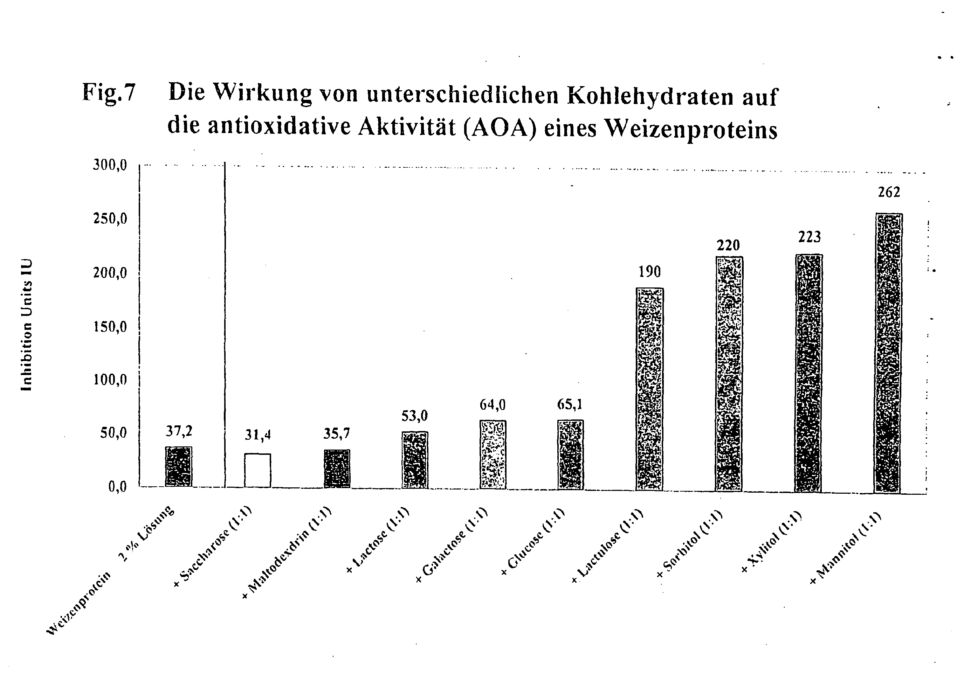

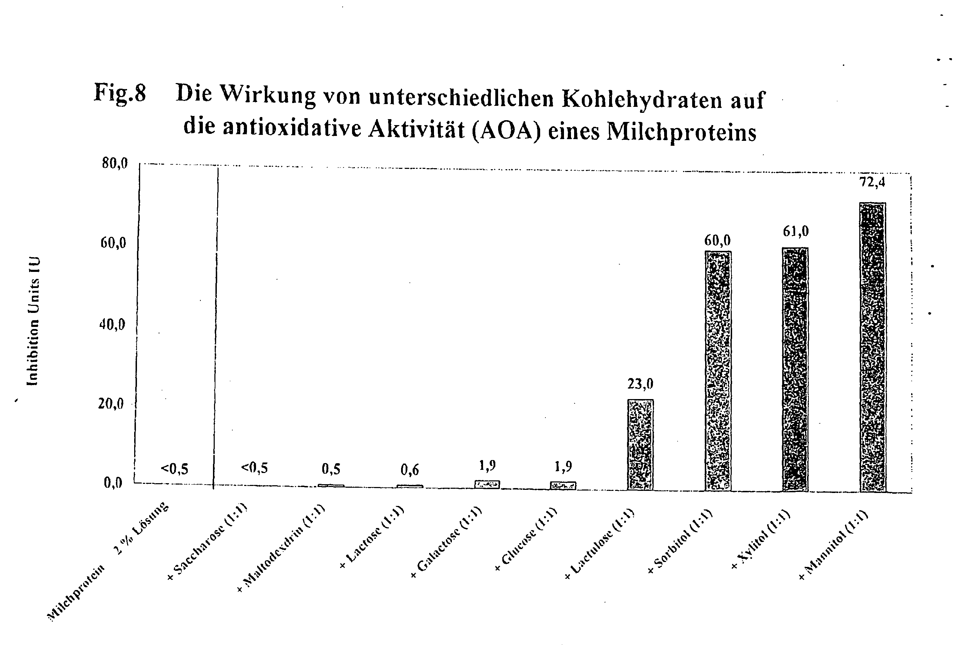

Die niedrigen Ursprungswerte der ausgewählten Kohlenhydrate [Fig. 4] bzw. der Einzelproteine [Fig. 5] zeigen bei entsprechender Kombinationskonzentration eine hoch signifikante Erhöhung der Gesamt- AOA-Werte, die weit über die Ursprungswerte der Proteine bzw. der Zucker gingen (+100 bis +400 %) [Fig. 6, Fig. 7, Fig. 8].

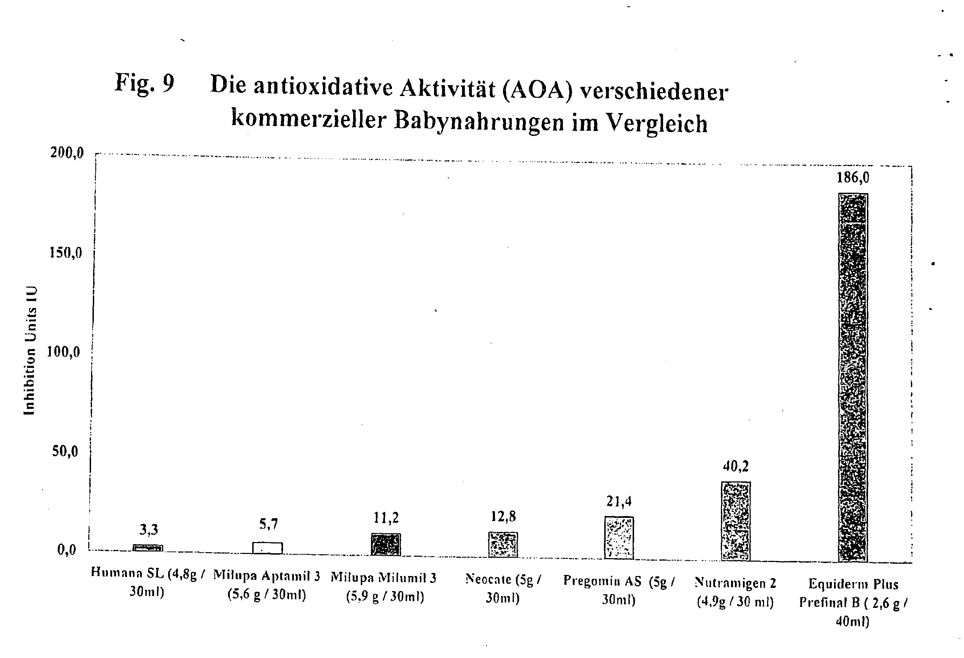

Die AOA-Werte eines vom Erfinder hergestellten diätetischen Lebensmittels, umfassend Molkeprotein und Sorbit als wirksame Protein-/Zuckermischung (Premix: "Equiderm Plus®";), schlägt eindeutig die antiinflammatorischen Eigenschaften herkömmlicher Babynahrungen [Fig. 9].

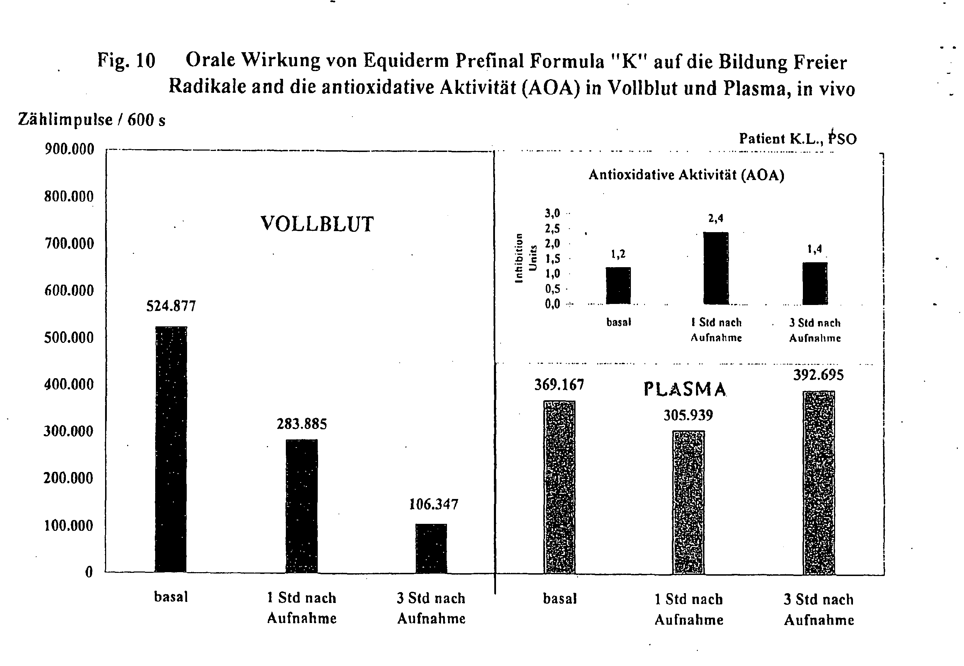

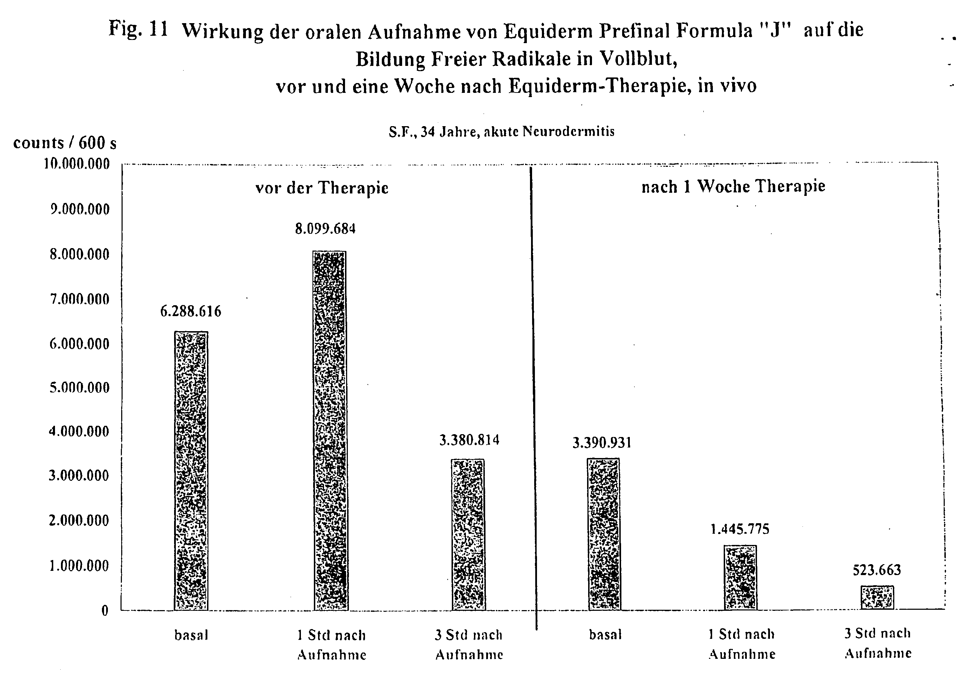

Die Verordnung solcher Mischungen beispielsweise an Neurodermitis-, Psoriasis-patienten führt schon über wenige Stunden zu einer prolongierten Hemmung der Produktion von freien Radikalen im Blut dieser Patienten, wie mit Hilfe der Chemoluminiszenz-Technologie nachweisbar ist [Fig. 10, Fig. 11], parallel mit einer spürbaren Reduzierung der klinischen Symptome (siehe auch Anwendungsstudie im Anhang).

Die antioxidative Aktivität (AOA) verschiedener biologischer Proben (Blut, Plasma, Proteinlösungen, Natursäfte, Equiderm, etc.) wurde mit Hilfe einer Freie Radikale erzeugenden Mischung von gepuffertem Lucigenin als Chemoluminiszenz-Substrat (1 ml) und Sodiumperborat (0,1 ml) (beides American Biologics, USA) bei einer Standardtemperatur von 36,5° C gemessen. Die Hemmung der Lichtemission durch eine biologische Probe wurde mit einem BJL Chemoluminiszenz-Analyzer (USA) während eines 5-minütigen AOA-Tests ermittelt. Eine AOA-Inhibitory Unit wurde als die 50%ige Reduktion der gesamten Lichtintensität der Lucigenin-Perborat-Quelle nach 5-minütiger Einwirkung von 94 $\mu$l einer 0,05 M L-Cystein Standardlösung definiert.

Zum Vergleich bzw. Berechnungsbeispiele siehe Literaturstelle 15.

Mischungen von Proteinen bzw. Proteinhydrolysaten mit erfindungsgemäßen Zuckerarten können in einem Verhältnis von 1:99 bis 99:1 vorliegen. Vorzugsweise beträgt das Verhältnis 1:2 bis 1:1.

Beispiel:

Konzentration der Einzelproteine in einer Tagesdosis von 1-10 g, einzeln oder in Kombination miteinander und Konzentration der Einzel-Zuckerarten in einer Tagesdosis von 1-10 g, einzeln oder in Kombination miteinander

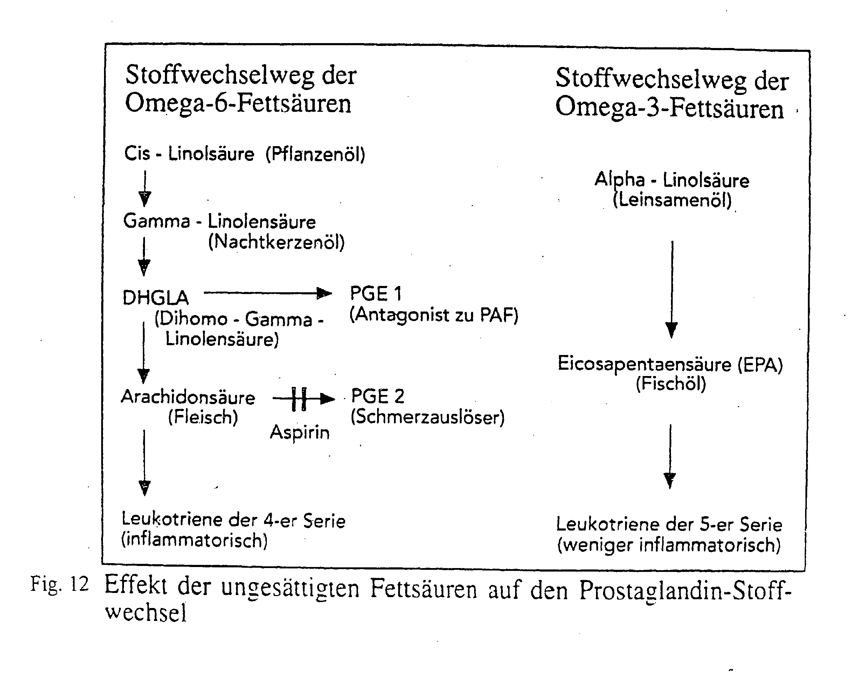

Mit Vorteil enthält das erfindungsgemäße Lebensmittel mehrfach ungesättigte Fettsäuren, insbesondere $\omega$-3-Fettsäuren. Beispiele ausgewählter $\omega$-3-Fettsäuren sind mehrfach ungesättigte Fettsäuren wie Eicosapentaen-säure (EPA), Docosahexaensäure (DHA) bzw. Alpha-Linolensäure mit synergistischer, steigernder freie Radikale bindende Wirkung im Blut der Probanden [Fig. 10, Fig. 11] und hemmendem Effekt auf den Syntheseweg der proinflammatorischen $\omega$-6-Arachidonsäure [Fig. 12].

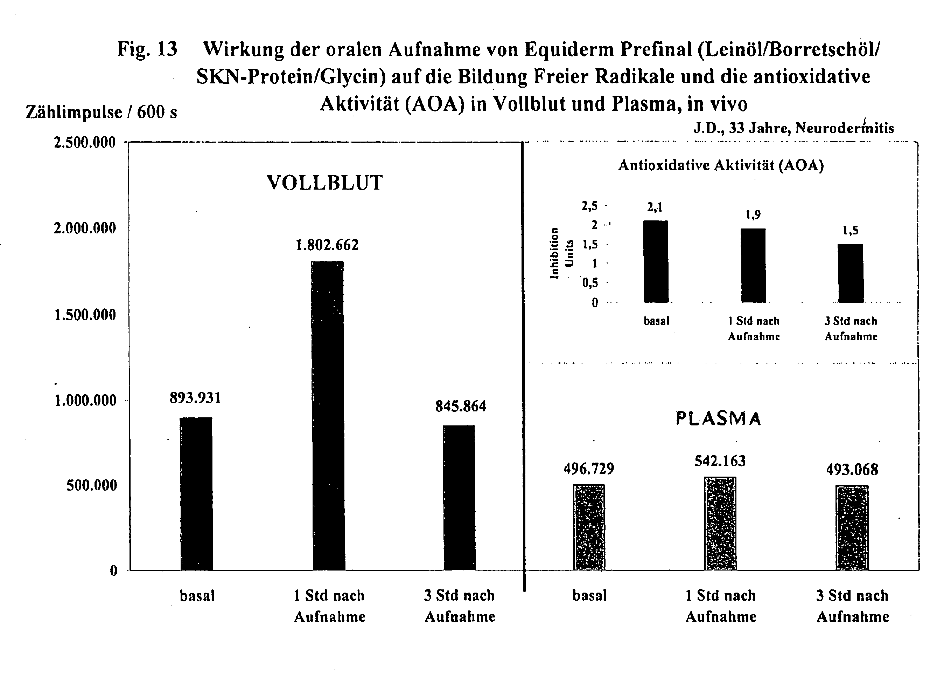

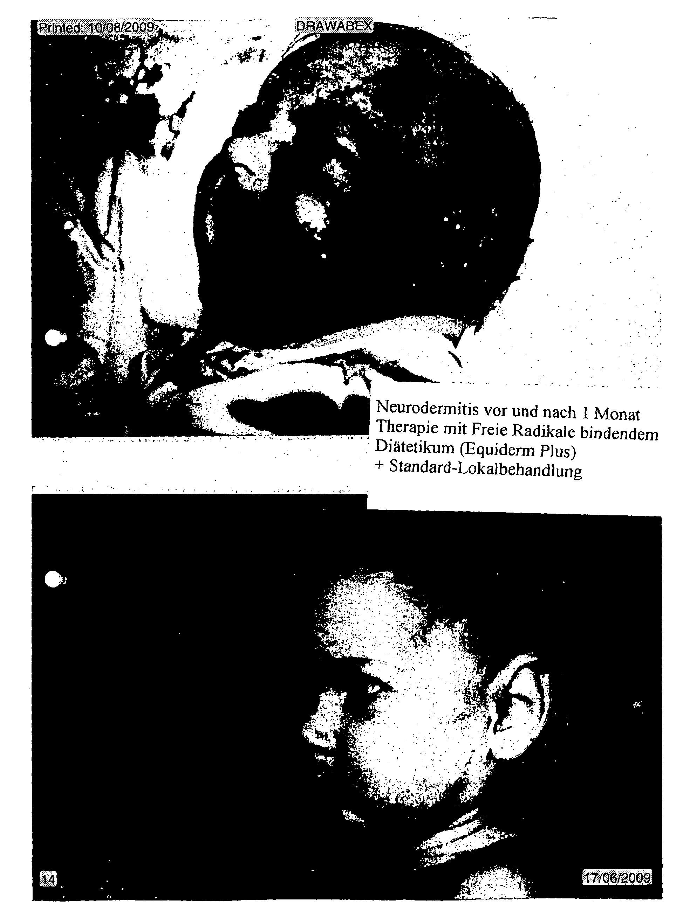

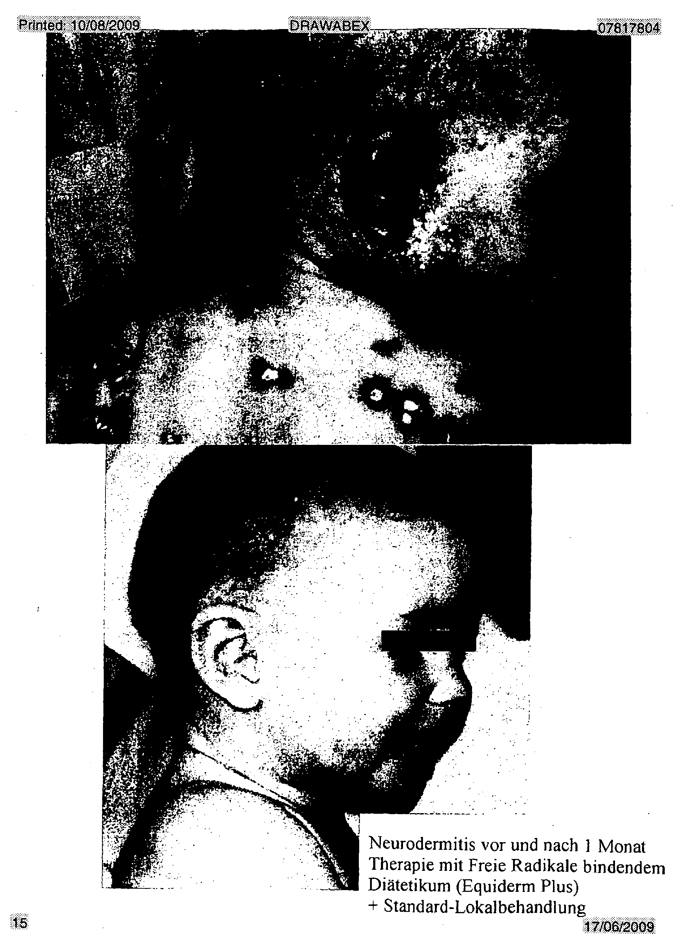

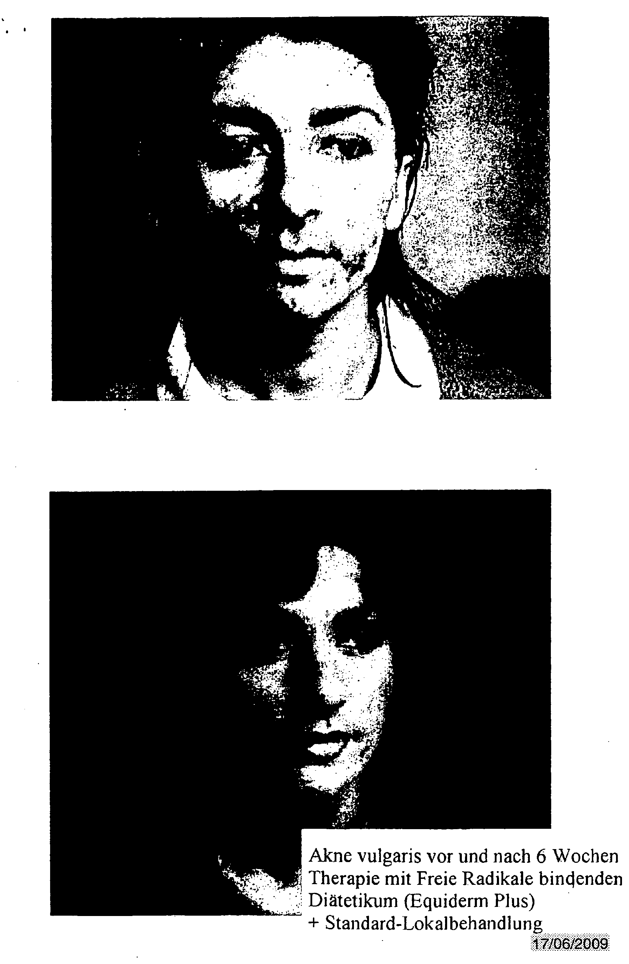

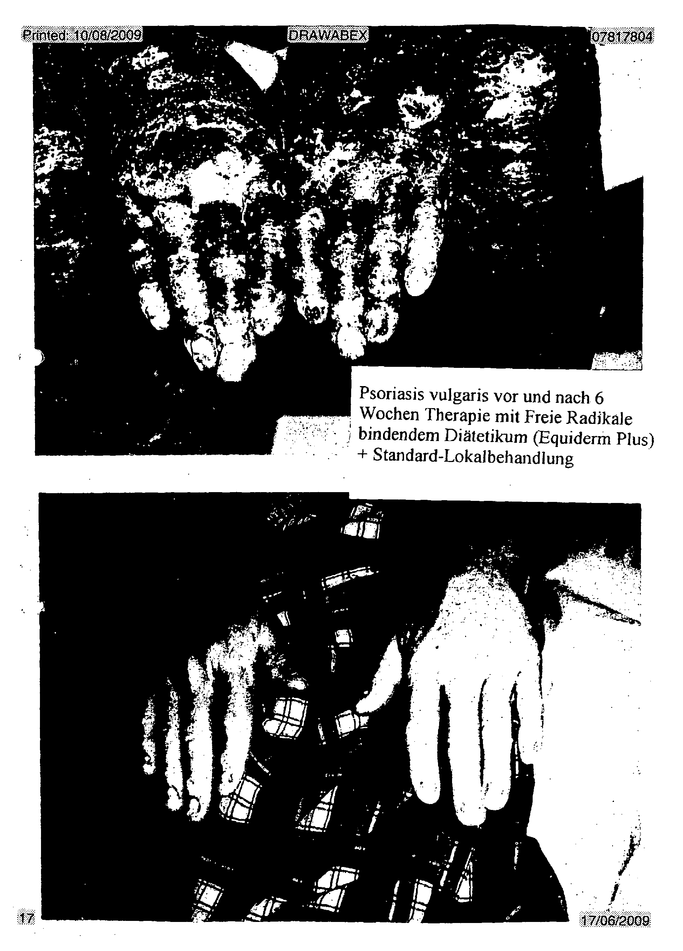

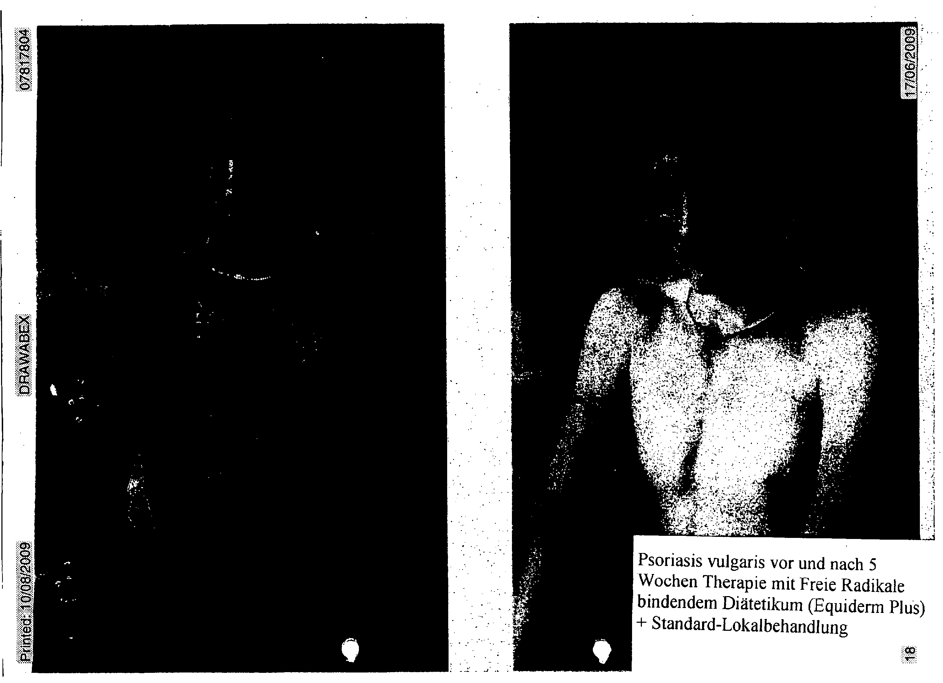

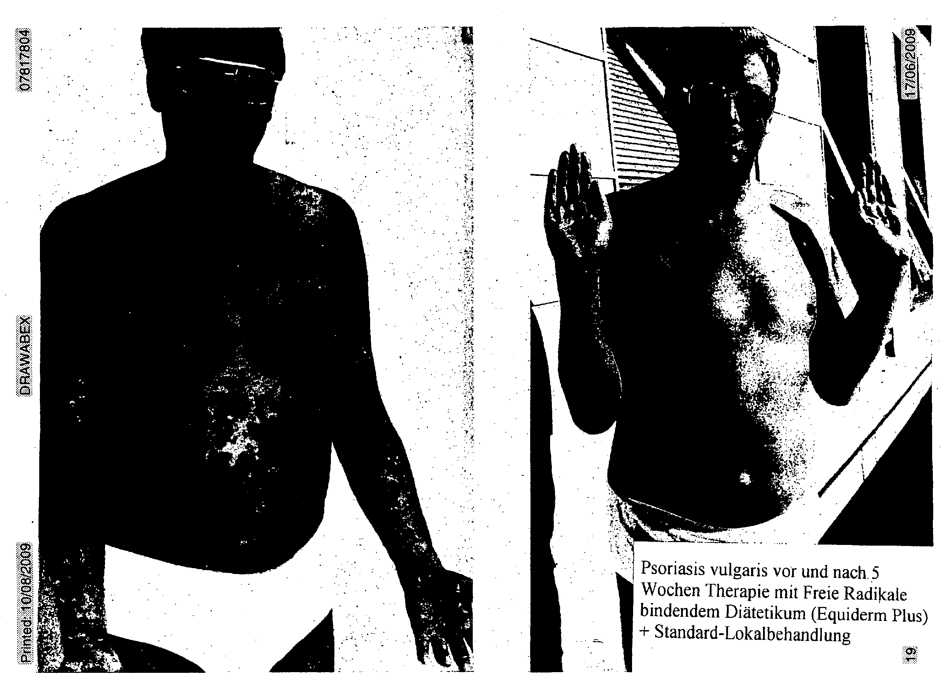

Umgekehrt haben die durchgeführten Tests immer wieder gezeigt, dass die Zugabe von $\omega$-6 enthaltenden Ölen wie Borretschöl, Distelöl oder Nachtkerzenöl zu einer Senkung der AOA-Wirkung führt [Fig. 13]. Diese Feststellung basiert im wesentlichen auf den bereits gestörten Gewichtsverhältnissen zwischen den $\omega$-6 und $\omega$-3 mehrfach ungesättigten Fettsäuren bei Neurodermitiskern und Psoriatikern, wie aus Fig. 1 und Fig. 2 zu entnehmen ist. Vorzugsweise ist daher das erfindungsgemäße Lebensmittel frei von $\omega$-6-Fettsäuren

Eine weitere Gabe von $\omega$-6 enthaltenden Ölen wie Borretsch-, Distel- oder Nachtkerzenöl verschlechtert das schon vorhandene Ungleichgewicht (10:1 bis 20:1) nochmals und begünstigt einen Ausbruch der Symptome, wie der Erfinder schon mehrmals beobachtet hat, parallel mit einer Steigerung der Produktion freier Radikale im Blut.

Aus diesem Grunde wird für die akut inflammatorischen Zustände verschiedener Dermatosen (Neurodermitis, Psoriasis, die diätetische Verwendung von $\omega$-3 mehrfach ungesättigten Fettsäuren wie Eicosapentaensäure (EPA), Docosahexaensäure (DHA) und / oder Alpha-Linolensäure (ALA) in adsorbierter Pulverform in Kombinationen mit der erfindungsgemäßen Protein-Zucker-Mischung zur synergistischen Steigerung der antiinflammatorischen Wirkung vorgeschlagen.

Beispiel:

Konzentration der ω-3-Fettsäuren in einer Tagesdosierung von 0,5 - 3 g, einzeln oder in Kombination miteinander

Die Kombination der genannten erfindungsgemäßen Protein-Zucker-Mischung mit ω-3-Fettsäuren (ALA, EPA, DHA) kann in einem Verhältnis von 1:99 bis 99:1 vorliegen.

Vorzugsweise weist das erfindungsgemäße diätetische Lebensmittel basische Elektrolyte, vorzugsweise in organischer Form, auf.

Die Oxidations- und Gärungsprozesse der Fettsäuren und Kohlenhydrate im Darm der Haut- und Umweltpatienten mit Bildung von Alkoholen, Aldehyden und organischen Säuren wie Essig-, Butter-, Propionsäure etc. führt zu einer chronisch latenten Azidose des Darmmilieus und des Bindegewebes. Zum Ausgleich dieser Si-tuation werden hier bevorzugt Elektrolyte mit basisch reduktiven Äquivalenten verwendet (Mg-Laktat, K3-Citrat bzw. tri-Ca-Citrat-Tetrahydrat). Die genannten organischen Elektrolytverbindungen besitzen einerseits eine ausgeprägte puffernde Kapazität saurer Äquivalente (niedriger pH-Wert im Darm, Blut und Bindegewebe), andererseits zeigen die Citratverbindungen eine gerinnungshemmende Wirkung im Blut und bewirken somit verbesserte Fließfähigkeiten des Blutes in den Kapillargefäßen, was zu einer höheren peripheren Durchblutung und $O_2$-Versorgung bei Allergien, Haut- und Autoimmunerkrankungen führt.

Beispiele:

Basische Elektrolyte aus Mg-, K-, Na- oder Ca-Laktat in einer Konzentration von je 0 bis 5 g Tagesdosis, einzeln oder in Kombination miteinander

Basische Elektrolyte aus Mg-, K-, Na- oder Ca-Citrat in einer Konzentration von je 0 bis 5 g Tagesdosis, einzeln oder in Kombination miteinander und
tri-Ca-Citrat-Tetrahydrat als basisches Elektrolyt in einer Tagesdosierung von 0-5 g

Konkretes Beispiel:

## Mg-Laktat 30 mg + K3-Citrat 18 mg + tri-Ca-Citrat-Tetrahydrat 30 mg

Vorzugsweise weist das erfindungsgemäße diätetische Lebensmittel freie Aminosäuren und/oder kurzkettige Fettsäuren mit Darmschleimhaut dichtender und aufbauender Wirkung, insbesondere Glutamin und/oder Na-Butyrat, auf.

In Kombination mit der erfindungsgemäßen Protein-Zucker-Mischung wird z.B. L-Glutamin als Aminosäure mit Darmschleimhaut dichtender Wirkung zusammen mit Natriumbutyrat verwendet.

Im Einklang mit den Literaturdaten und aufgrund der Erfahrungen des Erfinders bei Patienten mit einer erhöhten Darmdurchlässigkeit **[28]** besitzt L-Glutamin eine eindeutig aufbauende und abdichtende Wirkung auf die geschädigte Darmwand **[31, 32]**.

Vorzugsweise enthält das erfindungsgemäße diätetische Lebensmittel mindestens eine prebiotische Komponente, insbesondere Inulin, Laktulose, Fructooligosaccaridase (FOS) und/oder Galacto-Oligosaccharide (GOS).

Inulin (Alanstärke) ist ein Gemisch von Polysacchariden (Vielfachzuckern) und zählt zu den Fruktanen. Inulin wird in vielen Pflanzen als Reservestoff eingelagert. Beispiele sind Topinambur, Zichorien, Dahlie, Artischocke, Löwenzahn. Es ist leicht wasserlöslich und wird in der physiologischen Forschung zur Bestimmung des extrazellulären Raums eingesetzt, da es leicht in das Interstitium eindringt, jedoch nicht in die Zellen selbst. Inulin kann in der Therapie der Zuckerkrankheit (Diabetes mellitus) als Stärke-Ersatz eingesetzt werden, denn es wirkt nicht auf den Blutzucker-Spiegel ein. Inulin wird im Dünndarm nicht resorbiert, da dem Menschen das abbauende Enzym (Inulinase) fehlt. Stattdessen wird es im Enddarm von Bakterien zu kurzkettigen Fettsäuren abgebaut. Inulin dient vor allem den nützlichen Darmbakterien als Nahrung, regelmäßiger Verzehr führt zu einer Verbesserung der Darmflora.

Inulin und Oligofruktose werden heute zunehmend als funktionelle Lebensmittelzutaten (functional food) eingesetzt. Sie sind Ballaststoffe, die Magen und Dünndarm unverändert passieren. Erst im Dickdarm werden sie von den dort ansässigen Mikroorganismen abgebaut. Die Abbauprodukte fördern die erwünschten Milchsäurebakterien und schaffen ein für unerwünschte oder krankheitserregende Mikroorganismen ungünstiges Milieu. Prebiotika wie Inulin und Oligofruktose sollen Darminfektionen vorbeugen und das Immunsystem fördern **[33, 34]**.

Laktulose ist ein anderes Präbiotikum, welches als Grundnahrung für die physiologische Darmflora bei gleichzeitiger Reduzierung der Blutammoniakwerte dienen kann **[35]**.

Bei Laktulose-behandelten Ratten wurde eine signifikante Steigerung der Proliferation und Ansiedlungsfähigkeit festgestellt. In einer anderen Studie mit zirrhosekranken Ratten erhöhte die Laktulosebehandlung den Darmtransit und verbesserte die Darmdurchlässigkeit **[36]**.

Beispiel:

## Konzentrationen der Einzelprebiotika in einer Tagesdosis 0-15 g:

## einzeln oder in Kombination:  Inulin (0-10 g) + Lactulose (0-10 g) + GOS (0-10 g)

Vorzugsweise enthält das erfindungsgemäße diätetische Lebensmittel mindestens eine probiotische Komponente, insbesondere Milchsäure produzierende Bakterien, z.B. Lactobacillus reuteri.

Lactobacillus reuteri kann mit Vorteil beispielsweise in einer Tagesdosis von $0-10^{10}$ Bakterien/ g mit seinen darmflorastabilisierenden und immunmodulierenden Eigenschaften bei Allergien, Haut- und Autoimmunerkrankungen gegeben werden.

In der Praxis ist dem Erfinder bereits lange bekannt, dass Probiotika wie z.B. Laktobazillen das Gleichgewicht der Darmflora und die Immunfunktion des Darmes fördern **[37, 38]**.

Die eigenen klinischen Erfahrungen des Erfinders zeigen, dass eine 10-wöchige Lb. acidophilus-Verabreichung zu einer deutlichen Verbesserung der Obstipation, Flatulenz, der abdominalen Beschwerden und der nächtlichen Unruhen führte. Untersuchungen zur Prüfung der Überlebensrate des Lb. acidophilus im Darm von Patienten mit Neurodermitis 2 Monate nach Unterbrechung der Therapie werden derzeit durchgeführt. Die Zufuhr von Lb. acidophilus senkt die Aktivität der fäkalen, bakteriellen β-Glucuronidase, Azoreduktase und Nitroreduktase bei fleischreicher Ernährung. Diese Enzyme können azo- und aromatische Stickstoffverbindungen aus potentiell krankheitserregenden Substanzen reduzieren. Signifikante Zunahmen der Laktobazillenzahlen wurden bei Ergänzung der Diät mit Acidophilus-ND festgestellt. Die hohen Laktobazillenzahlen blieben erhalten, selbst wenn die zusätzliche Gabe von Acidophilus-ND beendet wurde. Die Aufnahme von VITAL DOPHILUS ® führte auch zu einer reduzierten Aktivität der fäkalen β-Glukosidase und β-Glukuronidase. Starterbakterien sind als ein bedeutender Lieferant von Antigenen zu betrachten, die zur Schulung des Abwehrsystems führen. Mit der Nahrung zugeführte Mikroorganismen sind offenbar gegenüber ihren Wirten stärker immunogen als die körpereigenen Organismen des entsprechenden Typs im Magen-Darmkanal **[37, 38]**.

Eine mit Lactobacillus reuteri ergänzte Babynahrung zeigte eine bessere Wirkung als eine Formel mit Bifidobacterium lactis (BB-12) bei der Bekämpfung chronischer intestinaler Infekte **[39]**.

Die vorliegende Erfindung betrifft ferner die Verwendung eines diätetischen Lebensmittels mit erhöhter antiinflammatorischer und freie Radikale bindender Wirkung, umfassend mindestens ein Protein und mindestens eine Zuckerart, wobei die freie Radikale bindende Wirkung der im Lebensmittel vorhandenen Protein-Zucker-Kombination um mindestens 50 % höher ist als die freie Radikale bindende Wirkung des einzelnen im Lebensmittel enthaltenen Proteins und/oder Zuckers, wobei das enthaltene Protein mindestens eines aus der Gruppe ist, bestehend aus Reisprotein, Weizenprotein, Erbsenprotein, Linsenprotein, Sojaprotein, Kartoffelprotein, Lupinenprotein, Molkeprotein, Collostrumprotein, Schafsmilchprotein, Ziegenmilchprotein, Stutenmilchprotein, und wobei der enthaltene Zucker mindestens einer aus der Gruppe ist, bestehend aus Xylit, Mannit, Maltit, Sorbit, Lactulose, zur Herstellung eines Mittels zur Behandlung von ällergisch bedingten Erkrankungen, augewählt aus Neurodermitis, Urtikaria, Milchschorf, allergisches Asthma, Psoriasis.

Die vorliegende Erfindung betrifft ferner die Verwendung des oben genannten Lebensmittels zur Behandlung von Psoriasis .

Zur Untermauerung der überragenden Wirksamkeit des erfindungsgemäßen Lebensmittels befindet sich im Anhang eine Anwendungsstudie zu einem Beispiel eines erfindungsgemäßen Lebensmittels (Equiderm plus®). Das bei diesem Lebensmittel verwendete Protein ist Molkeprotein, der wirksame Zuckerbestandteil ist Sorbit. Weitere Bestandteile sind Maltodextrin, Ballaststoffe (FOS), Inulin, Leinöl, Lecithin, L-Glutamin sowie verschiedene Vitamine und Spurenelemente. Bei der beiliegenden Anwendungsstudie wurde Patienten mit Neurodermitis und Psoriasis die genannte Zusammensetzung verabreicht.

Ferner liegen Vergleichsfotos bei, welche eindrucksvoll die überragende Wirksamkeit des genannten Präparates bei verschiedenen Erkrankungen zeigen.

**Literatur:**

1. Heller C.E.: Atopische Dermatitis im Kindesalter - Untersuchungen zum Aggressionsverhalten der Kinder und zum mütterlichen Erziehungsstil. Inaugural-Dissertation, Justus Liebig Universität Gießen, 2000.

2. Fairris G. et al: The Effect an Atopic Dermatitis of Supplementation with Selenium and Vitamin E. Acta Derm. Venerol. 69: 359-362, 1989.

3. Tsoureli N.E. et al: Evaluation of dietary intake of vitamin E in the treatment of atopic dermatitis: a study of the clinical course and evaluation of the immunoglobulin E serum levels. Int. J. Dermatol. 41 (3): 146-50, 2002.

4. el-Kohly M.S. et al: Zinc and copper status in childen with bronchial asthma and atopic dermatitis. J. Egypt. Public Health Assoc. 65 (5-6): 657-658, 1990.

5. Barth G.A. et al: Food intake of patients with atopic dermatitis. European Journal of Dermatology, 11 (3): 199-202, 2001.

6. Ionescu J.G. : Integrative Psoriasistherapie unter Berücksichtigung der Provokationsfaktoren. Derm 11, 59-64, 2005.

7. Andrew WF: The importance of the total load in the control of allergic symptoms. Clin Ebology 3, 145-148, 1987/88.

8. Stickl H: Umweltwirkungen auf das Immunsystem. Acta med Austriaca, 12, 6-16, 1985.

9. Prouvest-Danon et al: Induction of IgE synthesis and potentiation of antiovalbumin IgE antibody response by HgCl2 in the rat. J Immunol, 126, 699-702, 1981.

10. Ionescu G., Allergotoxische Einflüsse von Umweltschadstoffen bei Allergiekranken. Forsch. Komplementärmed. 2, 109-115, 1995.

11. Ionescu J.G.: Allergene, mikrobielle und Umweltfaktoren als Kausalitätstriade in der Pathogenese atopischer Erkrankungen. Bayer Vital Diagnostics AKTIV for you 8, 2006.

12. Arrigo AP, Kretz-Remy C.: Regulation of mammalian gene expression by free radicals. In: Aruoma O., Halliwell B. (eds.): Molecular biology of free radicals in human diseases, 183-216, 1998.

13. Ionescu J.G. et al.: Clinical application of free radical assessment in blood. ACAM Spring Conference Syllabus, Orlando, Florida, May 6-9, 1999. Reprint in: Townsend Letter, 197 (12), 102-104, 1999.

14. Ionescu G. et al.: Simple chemiluminescence assays for free radicals in venous blood and serum samples. Results in atopic, psoriasis, MCS and cancer patients. Forschende Komplementärmedizin, 6, 294-300, 1999.

15. Ionescu J.G. et al.: Clinical Application of Free Radical Assessment in Blood and Serum Samples by Enhanced Chemiluminescence. II. Antioxidative activity and therapy approaches with drugs and natural compounds. J. Biomed. Lab. Sci, 12, 46-56, 2000.

16. Ionescu G.: Clinical Application of Redox Potential Testing in the Blood. AAEM 33rd Annual Meeting, Baltimore, Syllabus, 503-512, 1998.

17. Ionescu J. G. et al.: Applications of redox and free radical assessment in the clinical practice. Int. J. of Gerontology, 3: 47-56, 2000.

18. Ionescu J.G.: Klinische Relevanz der Redox- und Chemoluminiszenzbestimmungen bei Allergien, Haut- und Umwelterkrankungen. In: Marktl, Reiter, Ekmekcioglu: Säuren, Basen, Schlacken, Springer-Verlag, 73-81, 2007.

19. Ionescu J.G.: Infant formulas for atopic children: selection criteria for protein hydrolysates. Communication at the Symposium "Aminosäuren in der Orthomolekularen Medizin, Prag, Feb. 2005.

20. Ionescu J.G. et al.: Optimaler Start ins Leben. Allergievorsorge beginnt schon im Mutterleib. Neurodermitis, 47, 6-7, 2006.

21. Yu G. et al.: Fatty acid composition in colostrum and mature milk from nonatopic and atopic mothers during the first 6 months of lactation. Acta Paediatr 87, 729-736, 1998.

22. Yu G. et Björksten B. : Polyunsaturated fatty acids in school children in relation to allergy and serum IgE levels. Pediatr Allergy Immunol, 9 (3), 133-138, 1998.

23. Ionescu G. et al.: Immunobiological Significance of Fungal and Bacterial Infections in Atopic Eczema. J. Adv. Med. (USA) 3 (1), 47-58, 1990.

24. Ionescu G. et al.: Abnormal fecal microflora and malabsorption phenomena in atopic eczema patients. J. Adv. Med. (USA) 3 (2), 71-91, 1990.

25. Salminen S. et al: Functional food science and gastrointestinal physiology and function. Br J Nutr 80, 147-171, 1998.

26. Gaskins HR: Immunological aspects of host / microbiota interactions at the intestinal epithelium. In: Mackie RI, While BA, Isaacson RE, eds. Gastrointestinal microbiology. New York. International Thomson Publishing 537-587, 1997.

27. Martinez FD, Holt PG: Role of microbial burden in aetiology of allergy and asthma. Lancet, 354, 12-15, 1999.

28. Ionescu G. et al.: Zirkulierende Immunkomplexe, spezifisches IgE gegen Nahrungsmittel- und Inhalationsallergene, Serumhistaminspiegel und Darmpermeabilitätsstörungen bei Neurodermitikern vor und nach Testmahlzeiten. Immun. Infekt. 13, 147-155, 1985.

29. Ionescu G.: Biologische Merkmale in der Pathogenese des chronischen Lupus Erythematodes Discoides. Dissertation, Universität des Saarlandes, Saarbrücken, 1983.

30. Ionescu G. et al: Serumkomplementfaktoren, Akute Phase Proteine und zirkulierende Immunkomplexe bei Neurodermitikern vor und nach Testmahlzeiten. Lab. Med. 10, 1-6, 1986.

31. Van der Berg A et al: The effect of glutamin-enriched enteral nutrition on intestinal permeability in very-low-birth- weight infants: a randomized controlled trial. JPEN J Parenter Enteral Nutr, 30, 408-414, 2006.

32. White J.S. et al: Glutamin improves intestinal barrier function in experimental biliary obstruction. Eur. Surg Res, 37 (6): 342-347, 2005.

33. Wade MA: Inulin and that "gut" feeling: inulin, a prebiotic typically found in dairy yogurts to enhance calcium absorption, also can provide moisture in meat substitutes and trade space with carbohydrates to supplement dietary fiber. Prepared Foods, July 2004.

34. de Wiele TV et al.: Prebiotic effects of chicory inulin in the simulator of the human intestinal microbial ecosystem. FEMS Microbiol Ecol, 51 (1), 143-153, 2004.

35. Dasarathy S.: Role of gut bacteria in the therapy of hepatic encephalopathy with lactulose and antibiotics. Indian J Gastroenterol Suppl. 2, S50-53, 2003.

36. Zhang S. et al: Effects of lactulose on intestinal endotoxin and bacterial translocation in cirrhotic rats. Chin Med J, 116 (5), 767-771, 2003.

37. Ionescu G. et al.: Oral lactobacilli can help manage atopic eczema. Dermatology News 21 (No. 3), p. 1, p. 10, 1988.

38. Ionescu G. et al.: Orale Laktobazillen bei atopischem Ekzem. Orthomolekular 4, 187-188, 1988.

39. Barclay L.: Probiotic infant formula may help reduce gastrointestinal tract infections CME. Pediatrics, 115, 5-9, 2005.

40: WO-A-03037320

41: WO-A-2006098625

42: JP-A - 2265458:

43:US-A-2006088574

44: EP-A-1340341

45:WO-A-01 44560

46: US-A-2001 197352

<div style="border: 2px solid black; text-align: center;">

# Equiderm Plus
# Eine Anwendungsbeobachtung bei Patienten mit Psoriasis und Neurodermitis

# Biometrischer Endbericht
# 18.04.2007

</div>

**Auswertung der Daten:**
i.A. MEDtest
Medical Consulting
Holzwiesengasse 15
A-3034 Maria Anzbach, Austria

**Biometrie / Datenmanagement:**
Mag.Dr. Judith Schuster

## Angaben zur Datenanalyse

Bei Equiderm Plus handelt es sich um ein ausgewogenes Nahrungsergänzungsmittel (ergänzende, bilanzierte Diät für besondere medizinische Zwecke - zur Behandlung atopischer Dermatitis, Psoriasis und Akne) das hypoallergene Proteine, mehrfach ungesättigte Fettsäuren und antioxidative Wirkstoffe enthält.

Die Wirkung des Präparates wurde von Mai bis November 2006 bei insgesamt 40 Hautpatienten beobachtet, von denen aber nur 30 Patienten statistisch ausgewertet werden konnten, da bei 10 Patienten keine PASI- bzw. SCOARD-Indices erhebbar waren. Bei den auswertbaren 30 Patienten waren 20 an Psoriasis und 10 an Neurodermitis erkrankt.

Die Behandlung der Patienten in der Spezialklink Neukirchen (Krankenhausstr., 93453 Neukirchen b. Hl. Blut) erfolgte während eines stationären Aufenthalts oder ambulant. Zur Dokumentation der Wirksamkeit wurden PASI (Psoriasis Area and Severity Index) und SCORAD (Europäische Expertengruppe für Atopische Dermatitis - adaptiert) Formulare vor Behandlungsbeginn und nach der 1-monatigen Therapie ausgefüllt. Für Informationen zur externen bzw. internen Therapie liegen Krankenberichte vor.

Alle Patienten die Equiderm Plus erhielten, bekamen keine orale Medikation. Es wurde ausschließlich die Haut extern behandelt. Die Neurodermitiker erhielten eine Herdsanierung, gefolgt von einer topischen Pflegebehandlung, bei den Psoriasis-Patienten wurden nach Salicylsäure-hältigen, keratolytische Salbenbehandlungen mit Dithranol durchgeführt.

Nach Angaben der Spezialklinik Neukirchen haben alle Patienten das Präparat gut vertragen und es waren keine Nebenwirkungen zu verzeichnen.

**Ziel der Anwendungsbeobachtung:**

Dokumentation von Veränderungen der Erkrankungen Psoriasis bzw. Neurodermitis nach Einnahme von Equiderm Plus.

**Der vorliegende Biometrische Bericht** enthält zusammenfassende Tabellen mit deskriptiver Darstellung der einzelnen Variablen. Die Änderungen in den PASI bzw. SCORAD Scores wurden ebenfalls graphisch dargestellt. Die Wirksamkeitsparameter PASI bzw. SCORAD wurden mit einem nichtparametrischen Test für verbunden Stichproben (Wilcoxon-Test) auf Veränderungen nach Einnahme von Equiderm Plus überprüft. Als Signifikanzniveau wurde $p < 0,05$ festgelegt.

Die Datenbank wurde in Microsoft Access 2003 erstellt und enthielt Formeln zur Berechnung der PASI bzw. SCORAD-Scores. So konnten Kalkulationsfehler auf den Formularen entdeckt und korrigiert werden (Pat.Nr.: 4, 6, 10,11,103,108,109,110). Die Analyse der Daten erfolgte in STATISTIKA/w 5.1. Die Graphiken wurden in Microsoft Excel 2003 erstellt.

**1. Patientencharakteristik - Demographische Daten**

| | Psoriasis | Neurodermitis |
|---|---|---|
| **Anzahl Patienten** (n) | 20 | 10 |
| **Alter** | | |
| Median (Min-Max), Jahre | 44 (17-71) | 44 (8-74) |
| **Geschlecht** | | |
| weiblich/männlich | 8 (40%) / 12 (60%) | 6 (6%) / 4 (4%) |
| **Zeitdifferenz Score 1 und 2** | | |
| Median (Min-Max), Tage | 27 (17-100) | 30 (21-60) |
| **Therapie intern** | | |
| Rotationstherapie (n) | 80% (16) | 90% (9) |
| Hepatoprotektive Therapie (n) | 10% (2) | - |
| Prednisolon f. 10 Tage (n) | 5%(1) | - |
| **Therapie extern** | | |
| Salicylsäurehältige Externa (n) | 55% (11) | Antibiotische/ |
| Dithranol (n) | 80% (16) | antiinflammatorische/ |
| LCD (n) | 60% (12) | antiphlogistische |
| Clotrimazol (n) | 20% (4) | Therapie, nicht näher definiert |
| **Ambulante Therapie** (n) | 20% (4) | 10% (1) |

**2.a. Psoriasis-Patienten - Detaillierte Statistik**

Der PASI-Fragebogen ist ein Messinstrument zur Beurteilung von Ausbreitung und Schweregrad der Psoriasis.

Der Körper wird in 4 Hautabschnitten (Kopf, Arme, Rumpf, Beine) beurteilt, die unterschiedlich gewichtet werden (10%, 20%, 30% 40%). Der prozentuellen Ausbreitung in jedem Hautabschnitt wird ein Punkte-Score zugeordnet, ebenso dem Schweregrad der Symptome Rötung, Hautschuppen und Hautdicke.

Die zeitliche Differenz zwischen den Auswertungen des Ansprechens (PASI 1 - PASI 2) liegt im Median relativ genau bei einem Monat (siehe 1.Patientencharakteristik), wobei jedoch auch größere bzw. kleinere Zeitspannen beobachtet wurden.

Zur Beschreibung der Verteilung werden einzelne gemessenen Variablen hier mit Mittelwert und Standardabweichung, Median sowie Minimum, Maximum, unterem und oberem Quartil dargestellt.

|  | n | Mittelwert | Median | Min | Max | Unteres Quartil | Oberes Quartil | SD |
|---|---|---|---|---|---|---|---|---|
| Alter | 20 | 42,20 | 44,09 | 17 | 71 | 29 | 55 | 16,66 |
| PASI-1 | 20 | 23,54 | 15,60 | 3,6 | 80 | 7,2 | 38,8 | 20,7 |
| PASI-2 | 20 | 3,01 | 2,15 | 0 | 10,8 | 1,15 | 4,5 | 2,5 |
| Prozent | 20 | 74,78 | 87,16 | 6,25 | 100 | 63,6 | 94,37 | 27,25 |
| Verbesserung Zeitdifferenz (Tage) | 20 | 36,8 | 27 | 17 | 100 | 24,5 | 38 | 22,76 |

**Altersverteilung Psoriasis-Patienten**

| Alter | Häufigkeit | Kumulative Häufigkeit | Prozent | Kumulativer Prozentwert |
|---|---|---|---|---|
| <20 | 3 | 3 | 15 | 15 |
| 21-30 | 3 | 6 | 15 | 30 |
| 31-40 | 3 | 9 | 15 | 45 |
| 41-50 | 5 | 14 | 25 | 70 |
| 51-60 | 2 | 16 | 10 | 80 |
| 61-70 | 3 | 19 | 15 | 95 |
| >70 | 1 | 20 | 5 | 100 |

|  | Auswertung 1 | Auswertung 2 |
|---|---|---|
| **Anzahl Patienten (n)** | 20 | 20 |
| **PASI Index** | | |
| Median (Min-Max) | 15,6 (3.6 - 80) | 2,15 (0 - 10,8) |
| Mittelwert (SD) | 23,5 (20,7) | 3,01 (2,5) |
| **Schweregrad** | | |
| Leicht (PASI <10) | 30% (n=6) | 95% (n=19) |
| Mittelschwer (PASI 10-50) | 55% (n=11) | 5% (n=11) |
| Schwer (PASI >50) | 15% (n=3) | 0 |

**Bei** 20 Patienten mit leichter bis schwerer Psoriasis ergab sich eine durchschnittliche Verbesserung des Hautzustandes um 75% nach 1 **Monat** Therapie mit Equiderm Plus.

**2.b. Graphische Darstellung der einzelnen PASI-Indexänderungen bzw. des Medians**

## 2.c. Signifikanz der Indexänderungen

Eine statistisch signifikante Verbesserung des PASI-Index durch die beschriebene Therapie (Wilcoxon-Test für paarige Stichproben, p<0,0001) kann beobachtet werden.

## 2.d. Auflistung von Einzelwerten

| Patno | Sex | Alter | PASI-1 | PASI-2 |
|---|---|---|---|---|
| 1 | w | 17 | 18,5 | 1,0 |
| 2 | w | 17 | 37,5 | 5,2 |
| 3 | w | 20 | 14,1 | 0,9 |
| 4 | m | 44 | 25,5 | 0,0 |
| 5 | w | 33 | 17,1 | 1,0 |
| 6 | w | 54 | 15,5 | 0,8 |
| 7 | m | 66 | 3,6 | 1,9 |
| 8 | m | 29 | 11,4 | 4,5 |
| 9 | m | 49 | 50,6 | 1,3 |
| 10 | m | 39 | 58 | 10,8 |
| 11 | m | 71 | 14,4 | 1,7 |
| 12 | w | 49 | 15,7 | 4,3 |
| 13 | m | 44 | 80 | 1,3 |
| 14 | m | 48 | 32,1 | 2,4 |
| 15 | w | 62 | 7,2 | 2,4 |
| 16 | m | 22 | 44 | 4,5 |
| 17 | w | 35 | 7,2 | 5,0 |
| 18 | m | 55 | 4,8 | 4,5 |
| 19 | m | 62 | 6,9 | 1,8 |
| 20 | m | 28 | 6,7 | 4,9 |

### 3. a. Neurodermitis Patienten - detaillierte Statistik

Der SCORAD-Fragebogen ist ein Messinstrument zur Beurteilung von Ausmaß und Intensität der Neurodermitis.

Der Index setzt sich zusammen aus dem prozentuellen Ausmaß von 10 Körperbereichen (Gesicht vorn, Kopf hinten, Obere Extremitäten vorn und hinten, Untere Extremitäten vorn und hinten, Rumpf vorn und hinten, Hände und Genitalien), die unterschiedlich gewichtet werden. Außerdem gehen in die Berechnung die Intensität verschiedener objektiver Symptome (Rötung, Schwellung, Nässen, Schuppung, Flechten, Hauttrockenheit) durch einen Punkte-Score und die subjektiven Symptome Juckreiz und Schlaflosigkeit (gemessen mit VAS) ein.

Der SCORAD-Index kann die Werte 0-103 (keine Neurodermitis - Ausbreitung über den ganzen Körper und sehr starke Symptome) annehmen.

Fehlende Angaben in einzelnen Bereichen wurden mit "0" bzw. "kein" angenommen, da es offensichtlich war, dass nur die betroffenen Hautbereiche ausgefüllt wurden.

Die zeitliche Differenz zwischen den Auswertungen des Ansprechens (SCORAD1 - SCORAD2) liegt im Median genau bei einem Monat (siehe 1. Patientencharakteristik), wobei jedoch auch größere bzw. kleinere Zeitspannen beobachtet wurden.

Zur Beschreibung der Verteilung werden einzelne gemessenen Variablen hier mit Mittelwert und Standardabweichung, Median sowie Minimum, Maximum, unterem und oberem Quartil dargestellt.

| | n | Mean | Median | Min | Max | Unteres Quartil | Oberes Quartil | SD |
|---|---|---|---|---|---|---|---|---|
| Alter | 10 | 42,87 | 44,41 | 8 | 74 | 34 | 60 | 21, 97 |
| SCORAD-1 | 10 | 75,92 | 87,7 | 37,6 | 101 | 48 | 96 | 24,25 |
| SCORAD-2 | 10 | 27,14 | 24,05 | 6,3 | 59,6 | 19 | 31 | 16,5 |
| Prozent Verbesserung | 10 | 65,66 | 68,11 | 37,9 | 86,9 | 59 | 75 | 14,23 |
| Zeitdifferenz (Tage) | 10 | 32,5 | 29,5 | 21 | 60 | 26 | 37 | 11,02 |

### Altersverteilung Neurodermitis-Patienten

| Alter | Häufigkeit | Kumulative Häufigkeit | Prozent | Kumulativer Prozentwert |
|-------|-----------|----------------------|---------|------------------------|
| <10 | 2 | 2 | 20 | 20 |
| 11-30 | 0 | 2 | 0 | 20 |
| 31-40 | 2 | 4 | 20 | 40 |
| 41-50 | 2 | 6 | 20 | 60 |
| 51-60 | 2 | 8 | 20 | 80 |
| 61-70 | 1 | 9 | 10 | 90 |
| >70 | 1 | 10 | 10 | 100 |

| | Auswertung 1 | Auswertung 2 |
|---|---|---|
| **Anzahl Patienten (n)** | **10** | **10** |
| **SCORAD Index** | | |
| Median (Min-Max) | 87,7 (37,6-101) | 24,05 (6,3-59,6) |
| Mittelwert (SD) | 75,92 (24,25) | 27,14 (16,5) |
| | | |
| **Schweregrad** | | |
| Leicht (SCORAD <25) | 0% (n=0) | 60% (n=6) |
| Mittelschwer (SCORAD 25-50) | 30% (n=3) | 20% (n=2) |
| Schwer (SCORAD >50) | 70% (n=7) | 20% (n=2) |

Bei 10 Patienten mit mittelschwerer bis schwerer Neurodermitis ergab sich eine durchschnittliche Verbesserung des Hautzustandes um 66% nach 1 Monat Therapie mit Equiderm Plus.

**3.b. Graphische Darstellung der einzelnen SCO-RAD-Index-änderungen bzw. des Medians**

### 3.c. Signifikanz der Indexänderungen

Eine statistisch signifikante Verbesserung des SCORAD-Index durch die beschriebene Therapie (Wilcoxon-Test für paarige Stichproben, p<0,01) kann beobachtet werden

### 3.d. Auflistung von Einzelwerten

| Patno | Sex | Alter | SCORAD1 | SCORAD2 |
|-------|-----|-------|---------|---------|
| 101 | m | 51 | 96,0 | 59,6 |
| 102 | w | 34 | 86,6 | 20,4 |
| 103 | m | 74 | 48,0 | 6,3 |
| 104 | w | 67 | 101,0 | 50,6 |
| 105 | w | 44 | 92,8 | 23,3 |
| 106 | m | 8 | 65,6 | 24,8 |
| 107 | w | 36 | 96,9 | 25,6 |
| 108 | w | 10 | 88,8 | 30,9 |
| 109 | m | 60 | 37,6 | 10,9 |
| 110 | w | 45 | 45,9 | 19,0 |

### 4. Zusammenfassung

Equiderm Plus, ein ausgewogenes Nahrungsergänzungsmittel, das hypoallergene Proteine, mehrfach ungesättigte Fettsäuren und antioxidative Wirkstoffe enthält, wurde von Mai bis November 2006 bei insgesamt 30 Hautpatienten in der Spezialklink Neukirchen (Krankenhausstr. 9, 93453 Neukirchen b. Hl. Blut) angewendet, die an Psoriasis (20 Patienten) bzw. Neurodermitis (10 Patienten) erkrankt waren.

Zur Dokumentation der Wirksamkeit wurden PASI (Psoriasis Area and Severity Index) und SCORAD (Europäische Expertengruppe für Atopische Dermatitis - adaptiert) Formulare vor Behandlungsbeginn und nach der 1-monatigen Therapie ausgewertet.

Nach einem Monat Therapie mit Equiderm Plus und reiner externer Hautbehandlung kam es bei 20 Patienten mit leichter bis schwerer Psoriasis zu einer signifikanten Verbesserung des Hautzustandes um 75%, bei 10 Patienten mit mittelschwerer bis schwerer Neurodermitis zu einer signifikanten Verbesserung des Hautzustandes um 66%.

**Patentansprüche**

1. Diätetisches Lebensmittel mit erhöhter antiinftammatorischer und freie Radikale bindender Wirkung zur Anwendung zur Behandlung von Neurodermitis, Urtikaria, Milchschorf, allergisches Asthma und/oder Psoriasis, umfassend mindestens ein Protein und mindestens eine Zuckerart, wobei die freie Radikale bindende Wirkung des Lebensmittels um mindestens 50 % höher ist als die freie Radikale bindende Wirkung des im Lebensmittel enthaltenen Proteins und/oder Zuckers, wobei das enthaltene Protein ausgewählt ist aus Reisprotein, Weizenprotein, Erbsenprotein, Linsenprotein, Sojaprotein, Kartoffelprotein, Lupinenprotein, Molkeprotein, Collostrumprotein, Schafsmilchprotein, Ziegenmilchprotein, Stutenmilchprotein, und der enthaltene Zucker ausgewählt ist aus Xylit, Mannit, Maltit, Sorbit, Lactulose, wobei das diätische Lebensmittel frei von Saccharose, Fructose und Laktose ist.

2. Diätetisches Lebensmittel nach Anspruch 1, **dadurch gekennzeichnet, dass** es bestimmte ungesättigte Fettsäuren, insbesondere Omega-3-Fettsäuren, enthält.

3. Diätetisches Lebensmittel nach einem der vorhergehenden Ansprüche, **dadurch gekennzeichnet, dass** es frei von Omega-6-Fettsäuren ist.

4. Diätetisches Lebensmittel nach einem der vorhergehenden Ansprüche, **dadurch gekennzeichnet, dass** es basische Elektrolyte, nämlich Mg-, K-, Na-oder Ca-Laktat oder Mg-, K-, Na- oder Ca-Citrat, aufweist.

5. Diätetisches Lebensmittel nach einem der vorhergehenden Ansprüche, **dadurch gekennzeichnet, dass** es freie Aminosäuren und/oder kurzkettige Fettsäuren mit Darmschleimhaut dichtender und aufbauender Wirkung, nämlich Glutamin und/oder Na-Butyrat, aufweist.

6. Diätetisches Lebensmittel nach einem der vorhergehenden Ansprüche, **dadurch gekennzeichnet, dass** es mindestens eine prebiotische Komponente, insbesondere Laktulose, Inulin, Fructooligosaccharide (FOS) und/oder Galacto-Oligosaccharide (GOS), enthält.

7. Diätetisches Lebensmittel nach einem der vorhergehenden Ansprüche, **dadurch gekennzeichnet, dass** es mindestens eine probiotische Komponente, insbesondere Milchsäure produzierende Bakterien, z.B. Lactobacillus reuteri, enthält.

8. Diätetisches Lebensmittel nach einem der vorhergehenden Ansprüche, **dadurch gekennzeichnet, dass** das enthaltene Protein hydrolysiert ist.

9. Verwendung eines diätetischen Lebensmittels mit erhöhter antiinflammatorischer und freie Radikale bindender Wirkung, umfassend mindestens ein Protein und mindestens eine Zuckerart, wobei die freie Radikale bindende Wirkung der im Lebensmittel vorhandenen Protein-Zucker-Kombination um mindestens 50 % höher ist als die freie Radikale bindende Wirkung des einzelnen im Lebensmittel enthaltenen Proteins und/oder Zuckers, wobei das enthaltene Protein mindestens eines aus der Gruppe ist, bestehend aus Reisprotein, Weizenprotein, Erbsenprotein, Linsenprotein, Sojaprotein, Kartoffelprotein, Lupinenprotein, Molkeprotein, Collostrumprotein, Schafsmilchprotein, Ziegenmilchprotein, Stutenmilchprotein, und wobei der enthaltene Zucker mindestens einer aus der Gruppe ist, bestehend aus Xylit, Mannit, Maltit, Sorbit, Lactulose, zur Herstellung eines Mittels zur Behandlung von Neurodermitis, Urtikaria, Milchschorf, allergisches Asthma und/oder Psoriasis.

**Claims**

1. Dietary foodstuff with increased anti-inflammatory and free-radical binding action for use in the treatment of neurodermatitis, urticaria, milk scall, allergic asthma and/or psoriasis, including at least one protein and at least one type of sugar, wherein the free-radical binding action of the foodstuff is at least 50% higher than the free-radical binding action of the protein and/or sugar present in the foodstuff, wherein the protein present is selected from rice protein, wheat protein, pea protein, lentil protein, soya protein, potato protein, lupin protein, whey protein, colostrum protein, sheep's milk protein, goat's milk protein, mare's milk protein, and the sugar present is selected from xylitol, mannitol, maltitol, sorbitol, lactulose, wherein the dietary foodstuff is free of sucrose, fructose and lactose.

2. Dietary foodstuff according to Claim 1, **characterized in that** it contains certain unsaturated fatty acids, in particular omega-3 fatty acids.

3. Dietary foodstuff according to one of the preceding claims, **characterized in that** it is free of omega-6 fatty acids.

4. Dietary foodstuff according to one of the preceding claims, **characterized in that** it has basic electrolytes, namely Mg, K, Na or Ca lactate or Mg, K, Na or Ca citrate.

5. Dietary foodstuff according to one of the preceding claims, **characterized in that** it has free amino acids and/or short-chain fatty acids, namely glutamine and/or Na butyrate, with a condensing and synthesizing effect on the mucous membrane.

6. Dietary foodstuff according to one of the preceding claims, **characterized in that** it contains at least one prebiotic component, in particular lactulose inulin, fructooligosaccharide (FOS) and/or galactooligosaccharide (GOS).

7. Dietary foodstuff according to one of the preceding claims, **characterized in that** it contains at least one probiotic component, in particular lactic acid-producing bacteria, e.g. Lactobacillus reuteri.

8. Dietary foodstuff according to one of the preceding claims, **characterized in that** the protein present is hydrolysed.

9. The use of a dietary foodstuff with increased anti-inflammatory and free-radical binding action, including at least one protein and at least one type of sugar, wherein the free-radical binding action of the protein-sugar combination present in the foodstuff is at least 50% higher than the free-radical binding action of the individual protein and/or sugar present in the foodstuff, wherein the protein present is at least one from the group consisting of rice protein, wheat protein, pea protein, lentil protein, soya protein, potato protein, lupin protein, whey protein, colostrum protein, sheep's milk protein, goat's milk protein, mare's milk protein, and wherein the sugar present is at least one from the group consisting of xylitol, mannitol, maltitol, sorbitol, lactulose, for the production of an agent for the treatment of neurodermatitis, urticaria, milk scall, allergic asthma and/or psoriasis.

**Revendications**

1. Aliment diététique à action accrue anti-inflammatoire et de fixation des radicaux libres, pour utilisation dans le traitement de la névrodermite, de l'urticaire, des croûtes de lait, de l'asthme allergique et/ou du psoriasis, comprenant au moins une protéine et au moins une type de sucre, l'action de fixation des radicaux libres présentée par l'aliment étant supérieure d'au moins 50 % à l'action de fixation des radicaux libres présentée par la protéine et/ou le sucre contenus dans l'aliment, la protéine contenue étant choisie parmi la protéine de riz, la protéine de blé, la protéine de pois, le protéine de lentille, la protéine de soja, la protéine de pomme de terre, la protéine de lupin, la protéine de lactosérum, la protéine de colostrum, la protéine de lait de brebis, la protéine de lait de chèvre, la protéine de lait de jument, et le sucre contenu étant choisi parmi le xylitol, le mannitol, le maltitol, le sorbitol, le lactulose, l'aliment diététique étant exempt de saccharose, fructose et lactose.

2. Aliment diététique selon la revendication 1, **caractérisé en ce qu'**il contient certains acides gras insaturés, en particulier des acides gras oméga-3.

3. Aliment diététique selon l'une quelconque des revendications précédentes, **caractérisé en ce qu'**il est exempt d'acides gras oméga-6.

4. Aliment diététique selon l'une quelconque des revendications précédentes, **caractérisé en ce qu'**il comporte des électrolytes basiques, à savoir du lactate de Mg, K, Na ou Ca ou du citrate de Mg, K, Na ou Ca.

5. Aliment diététique selon l'une quelconque des revendications précédentes, **caractérisé en ce qu'**il comporte des acides aminés libres et/ou des acides gras à courte chaîne à action restaurant et étanchéifiant la muqueuse intestinale, à savoir la glutamine et/ou le butyrate de Na.

6. Aliment diététique selon l'une quelconque des revendications précédentes, **caractérisé en ce qu'**il contient au moins un composant prébiotique, en particulier du lactulose, de l'inuline, des fructo-oligosaccharides (FOS) et/ou des galacto-oligosaccharides (GOS).

**7.** Aliment diététique selon l'une quelconque des revendications précédentes, **caractérisé en ce qu'**il contient au moins un composant probiotique, en particulier des bactéries productrices d'acide lactique, par exemple *Lactobacillus reuteri.*

**8.** Aliment diététique selon l'une quelconque des revendications précédentes, **caractérisé en ce que** la protéine contenue est hydrolysée.

**9.** Utilisation d'un aliment diététique à action accrue anti-inflammatoire et de fixation des radicaux libres, comprenant au moins une protéine et au moins une type de sucre, l'action de fixation des radicaux libres de l'association protéine-sucre présente dans l'aliment étant supérieure d'au moins 50 % à l'action de fixation des radicaux libres présentée par la protéine et/ou le sucre contenus dans l'aliment, la protéine contenue étant au moins une choisie dans le groupe constitué par la protéine de riz, la protéine de blé, la protéine de pois, le protéine de lentille, la protéine de soja, la protéine de pomme de terre, la protéine de lupin, la protéine de lactosérum, la protéine de colostrum, la protéine de lait de brebis, la protéine de lait de chèvre, la protéine de lait de jument, et le sucre contenu étant au moins l'un choisi dans le groupe constitué par le xylitol, le mannitol, le maltitol, le sorbitol, le lactulose, pour la fabrication d'un produit destiné au traitement de la névrodermite, de l'urticaire, des croûtes de lait, de l'asthme allergique et/ou du psoriasis.

# Fig. 1: Prozentuelle Verteilung der Fettsäuren in den Erythrozytenmembranen

Patient: A. R., 29 Jahre, männlich, Psoriasis vulgaris

**gesättigte Fettsäuren**

| | |
|---|---|
| Myristic | 0,3 |
| Palmitic | 23,1 |
| Stearic | 15,6 |
| Arachidic | 0,4 |
| Behenic | 1,7 |
| Tetracosanoic | 4,4 |

**einfach ungesättigte Fettsäuren**

| | |
|---|---|
| Myristoleic | 1,7 |
| Palmitoleic | 0,4 |
| Vaccenic | 1,1 |

**ω-3-Fettsäuren**

| | |
|---|---|
| Eicosapentaenoic | 0,2 |
| Docosahexaenoic | 2,4 |

**ω-6-Fettsäuren**

| | |
|---|---|
| gamma-Linolenic | |
| Linoleic | 8,6 |
| Dihomo-gamma-Linol. | 2,1 |
| Arachidonic | 16,6 |
| Adrenic | 2,9 |

**ω-9-Fettsäuren**

| | |
|---|---|
| Oleic | 9,9 |
| Gondoic | 0,3 |
| Nervonic | 4,3 |

0    5    10    15    20    25

Ratio ω-6 : ω-3 = 12:1

## Fig. 2: Prozentuelle Verteilung der Fettsäuren in den Erythrozytenmembranen

Patient: N. I., 46 Jahre, männlich, Psoriasis vulgaris

Ratio ω-6 : ω-3 = 14:1

**Fig.3  Die Wirkung unterschiedlicher Saccharosekonzentrationen auf die antioxidative Aktivität (AOA) von Molkeprotein**

Inhibition Units (IU)

| | 96,8 | 87,8 | 72,5 | 54,4 | 42,3 |

Molkeprotein 2 % Lösung  
+ 25 % Saccharose (4:1)  
+ 50 % Saccharose (2:1)  
+ 100 % Saccharose (1:1)  
+ 200 % Saccharose (1:2)

EP 2 097 089 B1

# Fig. 4 Die antioxidative Aktivität (AOA)
## von verschiedenen Kohlenhydraten

EP 2 097 089 B1

## Fig. 5 Antioxidative Aktivität (AOA) verschiedener Protein-Lösungen gemessen bei 36.5 °C durch Chemolumineszenz

jeweils 500 µl einer 2 % Proteinlösung

Antioxidative Units (IU)

Molkeproteinhydrolysat: 95,7
Weizenprotein: 37,2
Kuhmilchprotein: <0,5

Fig. 6    Die Wirkung von unterschiedlichen Kohlehydraten auf
die antioxidative Aktivität (AOA) des Molkeproteins

EP 2 097 089 B1

## Fig.7 Die Wirkung von unterschiedlichen Kohlehydraten auf die antioxidative Aktivität (AOA) eines Weizenproteins

**Fig.8    Die Wirkung von unterschiedlichen Kohlehydraten auf die antioxidative Aktivität (AOA) eines Milchproteins**

## Fig. 9 Die antioxidative Aktivität (AOA) verschiedener kommerzieller Babynahrungen im Vergleich

EP 2 097 089 B1

EP 2 097 089 B1

**Fig. 10** Orale Wirkung von Equiderm Prefinal Formula "K" auf die Bildung Freier Radikale and die antioxidative Aktivität (AOA) in Vollblut und Plasma, in vivo

## Fig. 11 Wirkung der oralen Aufnahme von Equiderm Prefinal Formula "J" auf die Bildung Freier Radikale in Vollblut, vor und eine Woche nach Equiderm-Therapie, in vivo

S.F., 34 Jahre, akute Neurodermitis

counts / 600 s

vor der Therapie — nach 1 Woche Therapie

| | vor der Therapie | | | nach 1 Woche Therapie | | |
|---|---|---|---|---|---|---|
| | basal | 1 Std nach Aufnahme | 3 Std nach Aufnahme | basal | 1 Std nach Aufnahme | 3 Std nach Aufnahme |
| counts | 6.288.616 | 8.099.684 | 3.380.814 | 3.390.931 | 1.445.775 | 523.663 |

EP 2 097 089 B1

EP 2 097 089 B1

Stoffwechselweg der
Omega-6-Fettsäuren

Stoffwechselweg der
Omega-3-Fettsäuren

Cis - Linolsäure  (Pflanzenöl)

↓

Gamma - Linolensäure
           (Nachtkerzenöl)

↓

DHGLA ────────► PGE 1
      (Dihomo - Gamma -      (Antagonist zu PAF)
      Linolensäure)

↓

Arachidonsäure ──╫──► PGE 2
(Fleisch)        Aspirin   (Schmerzauslöser)

↓

Leukotriene der 4-er Serie
(inflammatorisch)

Alpha - Linolsäure
(Leinsamenöl)

↓

Eicosapentaensäure (EPA)
(Fischöl)

↓

Leukotriene der 5-er Serie
(weniger inflammatorisch)

Fig. 12  Effekt der ungesättigten Fettsäuren auf den Prostaglandin-Stoffwechsel

**Fig. 13** Wirkung der oralen Aufnahme von Equiderm Prefinal (Leinöl/Borretschöl/
SKN-Protein/Glycin) auf die Bildung Freier Radikale und die antioxidative
Aktivität (AOA) in Vollblut und Plasma, in vivo

Zählimpulse / 600 s

J.D., 33 Jahre, Neurodermitis

EP 2 097 089 B1

Neurodermitis vor und nach 1 Monat
Therapie mit Freie Radikale bindendem
Diätetikum (Equiderm Plus)
+ Standard-Lokalbehandlung

Neurodermitis vor und nach 1 Monat
Therapie mit Freie Radikale bindendem
Diätetikum (Equiderm Plus)
+ Standard-Lokalbehandlung

Akne vulgaris vor und nach 6 Wochen
Therapie mit Freie Radikale bindenden
Diätetikum (Equiderm Plus)
+ Standard-Lokalbehandlung

Psoriasis vulgaris vor und nach 6
Wochen Therapie mit Freie Radikale
bindendem Diätetikum (Equiderm Plus)
+ Standard-Lokalbehandlung

Psoriasis vulgaris vor und nach 5 Wochen Therapie mit Freie Radikale bindendem Diätetikum (Equiderm Plus) + Standard-Lokalbehandlung

17/06/2009

Psoriasis vulgaris vor und nach 5
Wochen Therapie mit Freie Radikale
bindendem Diätetikum (Equiderm Plus)
+ Standard-Lokalbehandlung

17/06/2009

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03037320 A
- WO 2006098625 A
- JP 2265458 A
- US 2006088574 A
- EP 1340341 A
- WO 0144560 A
- US 2001197352 A


**In der Beschreibung aufgeführte Nicht-Patentliteratur**

- **Heller C.E.** Atopische Dermatitis im Kindesalter - Untersuchungen zum Aggressionsverhalten der Kinder und zum mütterlichen Erziehungsstil. *Inaugural-Dissertation,* 2000
- **Fairris G. et al.** The Effect an Atopic Dermatitis of Supplementation with Selenium and Vitamin E. *Acta Derm. Venerol.,* 1989, vol. 69, 359-362
- **Tsoureli N.E. et al.** Evaluation of dietary intake of vitamin E in the treatment of atopic dermatitis: a study of the clinical course and evaluation of the immunoglobulin E serum levels. *Int. J. Dermatol.,* 2002, vol. 41 (3), 146-50
- **el-Kohly M.S. et al.** Zinc and copper status in childen with bronchial asthma and atopic dermatitis. *J. Egypt. Public Health Assoc.,* 1990, vol. 65 (5-6), 657-658
- **Barth G.A. et al.** Food intake of patients with atopic dermatitis. *European Journal of Dermatology,* 2001, vol. 11 (3), 199-202
- **Ionescu J.G.** Integrative Psoriasistherapie unter Berücksichtigung der Provokationsfaktoren. *Derm,* 2005, vol. 11, 59-64
- **Andrew WF.** The importance of the total load in the control of allergic symptoms. *Clin Ebology,* 1987, vol. 3, 145-148
- **Stickl H.** Umweltwirkungen auf das Immunsystem. *Acta med Austriaca,* 1985, vol. 12, 6-16
- **Prouvest-Danon et al.** Induction of IgE synthesis and potentiation of antiovalbumin IgE antibody response by HgCl2 in the rat. *J Immunol,* 1981, vol. 126, 699-702
- **Ionescu G.** Allergotoxische Einflüsse von Umweltschadstoffen bei Allergiekranken. *Forsch. Komplementärmed.,* 1995, vol. 2, 109-115
- **Ionescu J.G.** Allergene, mikrobielle und Umweltfaktoren als Kausalitätstriade in der Pathogenese atopischer Erkrankungen. *Bayer Vital Diagnostics AKTIV for you 8,* 2006
- Regulation of mammalian gene expression by free radicals. **Arrigo AP ; Kretz-Remy C.** Molecular biology of free radicals in human diseases. 1998, 183-216
- **Ionescu J.G. et al.** Clinical application of free radical assessment in blood. ACAM Spring Conference Syllabus. *Townsend Letter,* 06. Mai 1999, vol. 197 (12), 102-104
- **Ionescu G. et al.** Simple chemiluminescence assays for free radicals in venous blood and serum samples. *Results in atopic, psoriasis, MCS and cancer patients. Forschende Komplementärmedizin,* 1999, vol. 6, 294-300
- **Ionescu J.G. et al.** Clinical Application of Free Radical Assessment in Blood and Serum Samples by Enhanced Chemiluminescence. II. Antioxidative activity and therapy approaches with drugs and natural compounds. *J. Biomed. Lab. Sci,* 2000, vol. 12, 46-56
- **Ionescu G.** Clinical Application of Redox Potential Testing in the Blood. *AAEM 33rd Annual Meeting,* 1998, 503-512
- **Ionescu J. G. et al.** Applications of redox and free radical assessment in the clinical practice. *Int. J. of Gerontology,* 2000, vol. 3, 47-56
- Klinische Relevanz der Redox- und Chemoluminiszenzbestimmungen bei Allergien, Haut- und Umwelterkrankungen. **Ionescu J.G.** Marktl, Reiter, Ekmekcioglu: Säuren, Basen, Schlacken. Springer-Verlag, 2007, 73-81
- **Ionescu J.G.** Infant formulas for atopic children: selection criteria for protein hydrolysates. *Communication at the Symposium "Aminosäuren in der Orthomolekularen Medizin,* Februar 2005
- **Ionescu J.G. et al.** Optimaler Start ins Leben. Allergievorsorge beginnt schon im Mutterleib. *Neurodermitis,* 2006, vol. 47, 6-7
- **Yu G. et al.** Fatty acid composition in colostrum and mature milk from nonatopic and atopic mothers during the first 6 months of lactation. *Acta Paediatr,* 1998, vol. 87, 729-736

- **Yu G. ; Björksten B.** Polyunsaturated fatty acids in school children in relation to allergy and serum IgE levels. *Pediatr Allergy Immunol,* 1998, vol. 9 (3), 133-138
- **Ionescu G. et al.** Immunobiological Significance of Fungal and Bacterial Infections in Atopic Eczema. *J. Adv. Med. (USA),* 1990, vol. 3 (1), 47-58
- **Ionescu G. et al.** Abnormal fecal microflora and malabsorption phenomena in atopic eczema patients. *J. Adv. Med. (USA),* 1990, vol. 3 (2), 71-91
- **Salminen S. et al.** Functional food science and gastrointestinal physiology and function. *Br J Nutr,* 1998, vol. 80, 147-171
- Immunological aspects of host / microbiota interactions at the intestinal epithelium. **Gaskins HR.** Gastrointestinal microbiology. International Thomson Publishing, 1997, 537-587
- **Martinez FD ; Holt PG.** Role of microbial burden in aetiology of allergy and asthma. *Lancet,* 1999, vol. 354, 12-15
- **Ionescu G. et al.** Zirkulierende Immunkomplexe, spezifisches IgE gegen Nahrungsmittel- und Inhalationsallergene, Serumhistaminspiegel und Darmpermeabilitätsstörungen bei Neurodermitikern vor und nach Testmahlzeiten. *Immun. Infekt.,* 1985, vol. 13, 147-155
- **Ionescu G.** Biologische Merkmale in der Pathogenese des chronischen Lupus Erythematodes Discoides. *Dissertation,* 1983
- **Ionescu G. et al.** Serumkomplementfaktoren, Akute Phase Proteine und zirkulierende Immunkomplexe bei Neurodermitikern vor und nach Testmahlzeiten. *Lab. Med.,* 1986, vol. 10, 1-6
- **Van der Berg A et al.** The effect of glutamin-enriched enteral nutrition on intestinal permeability in very-low-birth-weight infants: a randomized controlled trial. *JPEN J Parenter Enteral Nutr,* 2006, vol. 30, 408-414
- **White J.S. et al.** Glutamin improves intestinal barrier function in experimental biliary obstruction. *Eur. Surg Res,* 2005, vol. 37 (6), 342-347
- **Wade MA.** Inulin and that ″gut″ feeling: inulin, a prebiotic typically found in dairy yogurts to enhance calcium absorption, also can provide moisture in meat substitutes and trade space with carbohydrates to supplement dietary fiber. *Prepared Foods,* Juli 2004
- **de Wiele TV et al.** Prebiotic effects of chicory inulin in the simulator of the human intestinal microbial ecosystem. *FEMS Microbiol Ecol,* 2004, vol. 51 (1), 143-153
- **Dasarathy S.** Role of gut bacteria in the therapy of hepatic encephalopathy with lactulose and antibiotics. *Indian J Gastroenterol,* 2003, vol. 2, 50-53
- **Zhang S. et al.** Effects of lactulose on intestinal endotoxin and bacterial translocation in cirrhotic rats. *Chin Med J,* 2003, vol. 116 (5), 767-771
- **Ionescu G. et al.** Oral lactobacilli can help manage atopic eczema. *Dermatology News,* 1988, vol. 21 (3), 1, 10
- **Ionescu G. et al.** Orale Laktobazillen bei atopischem Ekzem. *Orthomolekular,* 1988, vol. 4, 187-188
- **Barclay L.** Probiotic infant formula may help reduce gastrointestinal tract infections CME. *Pediatrics,* 2005, vol. 115, 5-9